# EUROPEAN PATENT APPLICATION

(11) **EP 4 834 412 A1**
(43) Date of publication of application: **04.11.2026**
(21) Application number: 25748254.7
(22) Date of filing: 21.01.2025
(51) Int. Cl.: A61M 37/00

(54) **APPLICATOR**

(30) Priority: 29.01.2024 JP 2024011295
(71) Applicant: Hisamitsu Pharmaceutical Co., Inc., Tosu-shi, Saga 841-0017 (JP)
(72) Inventor: ARAI, Yuta, Tosu-shi, Saga 841-0023 (JP); MATSUDO, Toshiyuki, Tosu-shi, Saga 841-0023 (JP); NISHIMURA, Shinpei, Tosu-shi, Saga 841-0023 (JP)
(74) Representative: Grünecker Patent- und Rechtsanwälte PartG mbB
(86) International application number: PCT/JP2025/001784
(87) International publication number: WO 2025/164446

(57) **Abstract**

The applicator is an applicator for applying a microneedle device to skin, including: a main body including a bottom portion in contact with the skin, a top portion opposite to the bottom portion, and a side portion that extends in a height direction from the bottom portion toward the top portion; a switch including an arm mounted on the side portion to extend in a lateral direction from the side portion toward a center of the main body, and a piston provided at a free end of the arm and pushed in by a user toward the skin; and at least one stopper that supports the microneedle device disposed in the main body to be located between the piston and the skin.

## Description

### Technical Field

One aspect of the present disclosure relates to an applicator.

### Background Art

Patent Literature 1 describes an application device including a housing and an impactor that impacts a microneedle array to accelerate microneedles toward a target site. Patent Literature 2 describes a device that includes a microneedle array and a housing in which the microneedle array is disposed and which defines a chamber, and in which the microneedles penetrate a surface of a body. Patent Literature 3 describes a microneedle-based transdermal drug delivery device for administering a liquid drug to a user's skin.

### Citation List

### Patent Literature

[Patent Literature 1] Japanese Unexamined Patent Publication No. 2008-543527
[Patent Literature 2] Japanese Unexamined Patent Publication No. 2017-509449
[Patent Literature 3] United States Patent Application, Publication No. 2013/0296824

### Summary of Invention

### Technical Problem

An applicator that can properly apply the microneedle device to the skin is desired.

### Solution to Problem

An applicator in accordance with an aspect of the present disclosure is an applicator for applying a microneedle device to skin, including a main body including a bottom portion in contact with the skin, a top portion opposite to the bottom portion, and a side portion that extends in a height direction from the bottom portion toward the top portion, a switch including an arm mounted on the side portion to extend in a lateral direction from the side portion toward a center of the main body, and a piston provided at a free end of the arm and pushed in by a user toward the skin, and at least one stopper that supports the microneedle device disposed in the main body to be located between the piston and the skin.

In this aspect, a microneedle device disposed in the main body to be located between the piston and the skin is supported by at least one stopper. Thus, when a user pushes the piston toward the skin, the piston can be brought into stable contact with the microneedle device. As a result, the microneedle device is pushed reliably toward the skin. Thus, the microneedle device can be properly applied to the skin.

### Advantageous Effects of Invention

According to an aspect of the present disclosure, the microneedle device can be properly applied to the skin.

### Brief Description of Drawings

FIG. 1 is a plan view of an applicator according to a first example, seen from above.
FIG. 2 is a perspective cross-sectional view along line II-II shown in FIG. 1.
FIG. 3 is a perspective view of the applicator according to the first example, seen from below.
FIG. 4 is a diagram showing a process until a microneedle device is applied to skin.
FIG. 5 is a perspective view of an application kit according to the first example, seen from above.
FIG. 6 is a cross-sectional view along line VI-VI shown in FIG. 5.
FIG. 7 is a diagram showing a process until the microneedle device is disposed in the applicator.
FIG. 8 is a plan view of an applicator according to a second example, seen from above.
FIG. 9 is a perspective cross-sectional view along line IX-IX shown in FIG. 8.
FIG. 10 is a perspective view of the applicator according to the second example, seen from below.
FIG. 11 is a diagram showing a process until a microneedle device is applied to skin.
FIG. 12 is a perspective view of an application kit according to the second example, seen from above.
FIG. 13 is a cross-sectional view along line XIII-XIII shown in FIG. 12.
FIG. 14 is a plan view of an applicator according to a third example, seen from above.
FIG. 15 is a perspective cross-sectional view along line XV-XV shown in FIG. 14.
FIG. 16 is a perspective cross-sectional view along line XVI-XVI shown in FIG. 14.
FIG. 17 is a diagram showing a state in which a microneedle device is set in the applicator.
FIG. 18 is a diagram showing a process until gripping claws grip the microneedle device.
FIG. 19 is a perspective view of an application kit according to the third example, seen from above.
FIG. 20 is a plan view of an applicator according to a fourth example, seen from above.
FIG. 21 is a perspective cross-sectional view along line XXI-XXI shown in FIG. 20.
FIG. 22 is a diagram showing a state in which a microneedle device is set in the applicator.
FIG. 23 is a perspective view of an application kit according to the fourth example, seen from above.
FIG. 24 is a plan view of an applicator according to a fifth example, seen from above.
FIG. 25 is a perspective cross-sectional view along line XXV-XXV shown in FIG. 24.
FIG. 26 is a perspective view of an application kit according to the fifth example, seen from above.
FIG. 27 is a plan view of an applicator according to a sixth example, seen from above.
FIG. 28 is a perspective cross-sectional view along line XXVIII-XXVIII shown in FIG. 27.
FIG. 29 is a perspective view of an application kit according to the sixth example, seen from above.

### Description of Embodiments

Hereinafter, various examples in the present disclosure are described in detail with reference to the accompanying drawings. In the description of the drawings, identical or equivalent elements are designated by the same symbol, and redundant descriptions thereof will be omitted.

### [Overview]

An applicator according to the present disclosure is an auxiliary tool used to apply a microneedle device to the skin for administering any active ingredient (for example, a drug) into a living body. The applicator allows a user to apply the microneedle device to the skin with more appropriate force than when the microneedle device is directly applied with their hands. Applying a microneedle device to the skin means pushing the microneedle device toward the skin by manipulation of the applicator by the user, thereby causing one or more microneedles provided on the microneedle device to be in contact with or pierce the skin.

The microneedle device to be applied to the skin is set in the applicator. In the present disclosure, a state in which the microneedle device to be applied to the skin is set in the applicator is also referred to as a set state. A state in which the microneedle device is not set in the applicator is also referred to as a natural state.

In one example, the applicator is provided as a component of an application kit. The application kit has an applicator and a stage for setting the microneedle device in the applicator. The application kit may be further equipped with a microneedle device.

### [Applicator According to First Example]

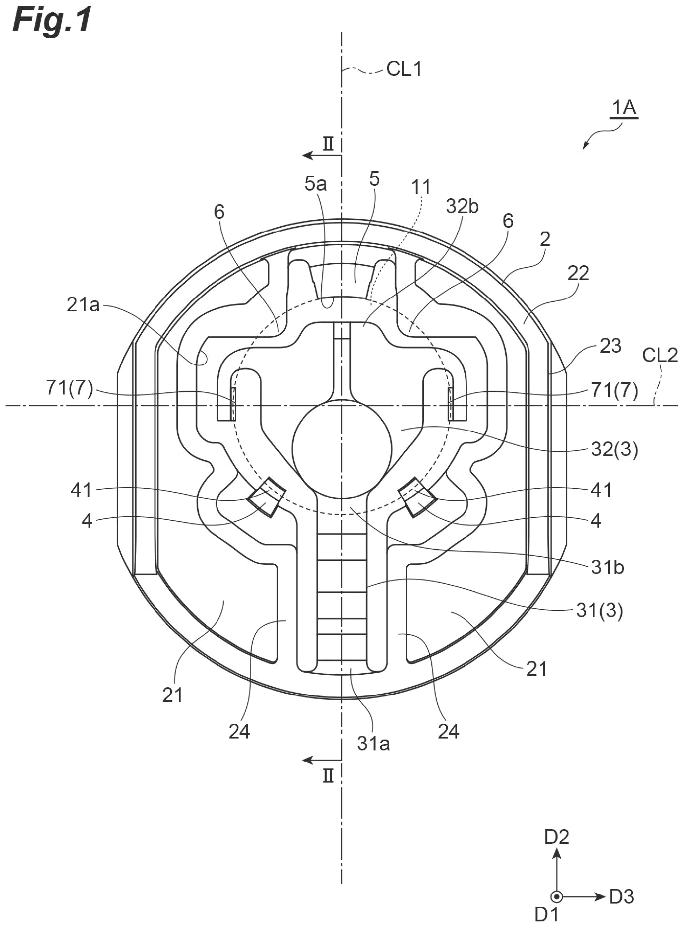

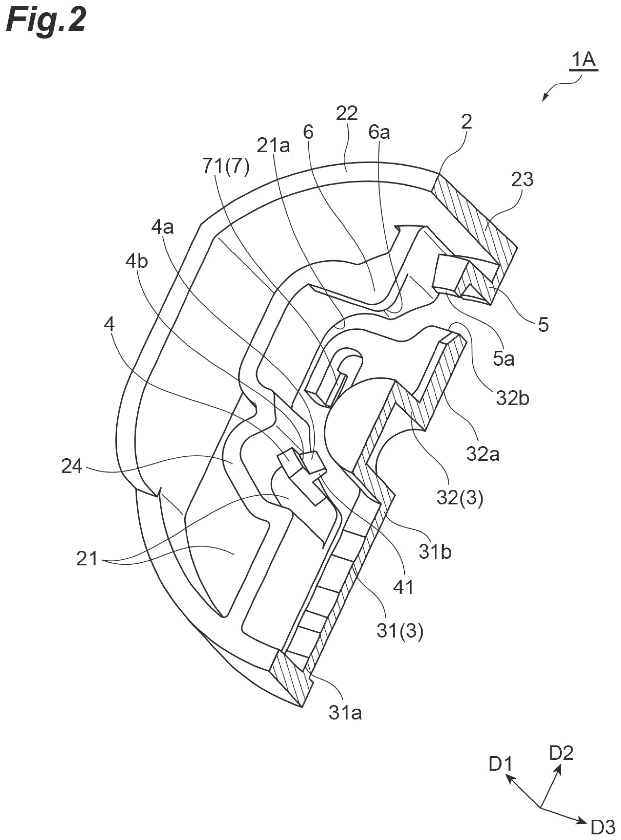

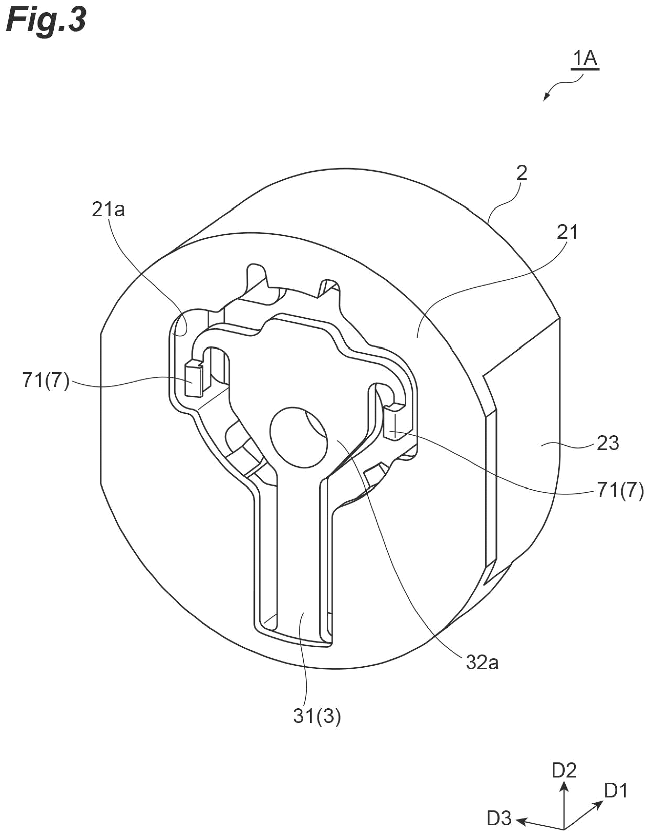

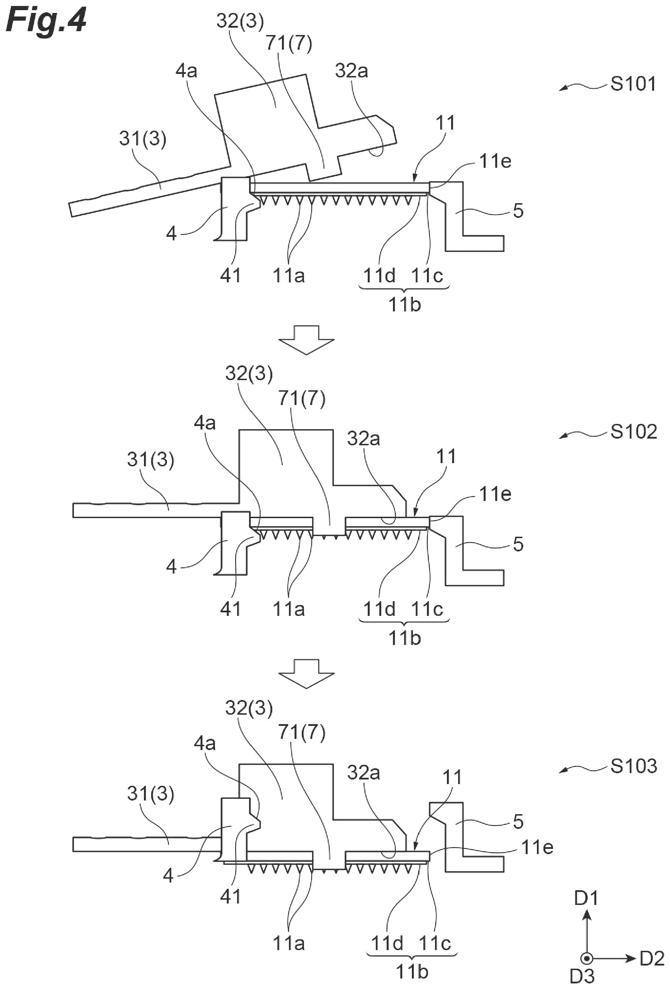

A structure of an applicator 1A according to a first example is described with reference to FIGS. 1 to 4. FIG. 1 is a plan view of the applicator 1A seen from above. FIG. 2 is a perspective cross-sectional view along line II-II shown in FIG. 1. FIG. 3 is a perspective view of the applicator 1A seen from below. FIG. 4 is a diagram showing a process from the state in which the microneedle device is set in the applicator 1A to when the microneedle device is applied to the skin. FIGS. 1 to 3 show the applicator 1A in the natural state.

The applicator 1A includes a main body 2, a switch 3, at least one stopper 4, at least one rib 5, at least one eaves portion 6, and a recovery mechanism 7. A material of the applicator 1A is a synthetic or natural resin material, such as ABS resin, polystyrene, polypropylene, polyacetal (POM), polycarbonate, polybutylene terephthalate, polyamide, polyetheretherketone, polyethylene, polyethylene terephthalate, or the like. The material of the applicator 1A may be silicon, silicon dioxide, ceramic, or a metal (stainless steel, titanium, nickel, molybdenum, chromium, cobalt, or the like). Alternatively, glass fibers or carbon fibers may be added to the above resin material to further increase a strength of the applicator 1A. The material of the applicator 1A may be a mixture of two or more of the above materials. As an example, the applicator 1A is formed in one piece by introducing the above materials into a mold.

As shown in FIGS. 1 and 4, the microneedle device 11 has a circular shape. The shape of the microneedle device 11 may be any shape, such as a square, oval, crescent, or other polygonal shape. In FIG. 1, an outer edge of the microneedle device 11 in a set state is indicated by a dotted line. The microneedle device 11 includes one or more microneedles 11a and a main surface 11b that supports the one or more microneedles 11a. The main surface 11b has a peripheral edge portion 11c that defines the outer edge of the microneedle device 11, and a platform surface 11d that is formed inside the peripheral edge portion 11c at a position raised from the peripheral edge portion 11c and supports the one or more microneedles 11a. The platform surface 11d is an octagonal surface located inward with respect to the outer edge of the microneedle device 11. The shape of the platform surface 11d may be any shape, such as a square, circle, oval, crescent, or other polygonal shape. The microneedles 11a are provided on the platform surface 11d and not provided on the peripheral edge portion 11c. The microneedle device 11 further has a side surface 11e that extends in thickness and circumferential directions of the microneedle device 11. The side surface 11e coincides with the outer edge of the microneedle device 11 when seen in the thickness direction of the microneedle device 11. The peripheral edge portion 11c is a portion that includes the side surface 11e.

The main surface 11b does not have to have a platform surface 11d. In this case, the main surface 11b is a uniformly flat surface that does not have a stepped structure that distinguishes the peripheral edge portion 11c from the platform surface 11d, and the microneedles 11a are provided on the flat surface.

The material of the microneedle device 11 may be, for example, silicon, silicon dioxide, ceramic, a metal (stainless steel, titanium, nickel, molybdenum, chromium, cobalt, or the like), or a synthetic or natural resin material. Alternatively, in consideration of antigenicity of the microneedles 11a and unit cost of the materials, biodegradable polymers such as polylactic acid, polyglycolide, polylactic acid-co-polyglycolide, pullulan, caprolactone, polyurethane, and polyanhydride may be used, or a synthetic or natural resin material which is non-degradable polymers, such as polyethylene, polypropylene, polyamide, polyethylene terephthalate, cyclic olefin copolymers, polycarbonate, polymethacrylic acid, polymethyl methacrylate, rigid polyvinyl chloride, ethylene vinyl acetate, polytetrafluoroethylene, tetrafluoroethylene-ethylene copolymer, polyoxymethylene, acrylonitrile-butadiene-styrene copolymer, polyetheretherketone, and polystyrene may be used. Alternatively, liquid crystal polymers such as thermotropic liquid crystal polymers may be used. Alternatively, polysaccharides such as hyaluronic acid, sodium hyaluronate, pullulan, dextran, dextrin or chondroitin sulfate, and cellulose derivatives may be used. In consideration of a possibility that the microneedles 11a may break on the skin, biodegradable resin may be used, for example, polylactic acid. Polylactic acid includes polylactic acid homopolymers such as poly-L-lactic acid and poly-D-lactic acid, poly-L/D-lactic acid copolymers, and mixtures thereof, and any of them may be used. As an average molecular weight of the polylactic acid increases, the strength thereof becomes stronger, and, for example, polylactic acid with an average molecular weight of 40,000 to 100,000 may be used. Portions of the microneedle device 11 other than the microneedles 11a, and the microneedles 11a may be manufactured of the same material, or of different materials.

The main surface 11b is a base for supporting the microneedles 11a. An area of the main surface 11b may be 0.5 cm² to 300 cm², 1 cm² to 100 cm², or 1 cm² to 50 cm². Alternatively, the area of the main surface 11b may be 0.5 cm² to 10 cm², 0.5 cm² to 5 cm², 1 cm² to 5 cm², 0.5 cm² to 3 cm², or 1 cm² to 3 cm². A microneedle device having a desired size may be formed by connecting several microneedle devices 11 together. A lower limit of a thickness of the peripheral edge portion 11c may be 5 µm, 20 µm, 50 µm, 300 µm, or 500 µm, and an upper limit of the thickness may be 2000 µm, 1500 µm, 1200 µm, 1000 µm, or 800 µm.

Each of the microneedles 11a is a tiny structure that stands up from the platform surface 11d. The microneedle 11a has a tapered shape that tapers from a bottom portion, at which it is in connect with the platform surface 11d, to a tip end portion. In the specification, the term "microneedle" is a concept that includes not only needle-shaped structures and structures that include a needle shape in a broad sense, but also non-pointed shapes.

A lower limit of a density of the microneedles 11a may be, for example, 0.05 needles/cm², 1 needle/cm², 50 needles/cm², 75 needles/cm², 100 needles/cm², 150 needles/cm², 200 needles/cm², 300 needles/cm², 340 needles/cm², or 400 needles/cm². On the other hand, an upper limit of the density may be, for example, 10,000 needles/cm², 5,000 needles/cm², 2,000 needles/cm², 1,200 needles/cm², 700 needles/cm², 640 needles/cm², or 340 needles/cm². The lower limit of the density is a value calculated from the number of microneedles capable of administering 1 mg of an active ingredient and a required area. The upper limit of the density is a limit value that is determined by taking into consideration the shape of the microneedles.

A lower limit of a length of the microneedles 11a may be 20 µm, 50 µm, 150 µm, 200 µm, or 300 µm, and an upper limit thereof may be 1200 µm, 900 µm, or 500 µm. The length of the microneedles 11a is a distance from the bottom portion, at which it is in connect with the platform surface 11d, to the tip end. The reason why the length of the microneedles 11a is set to 20 µm or more is to ensure transdermal absorption of the active ingredient. By setting the length of the microneedles 11a to 900 µm or less, it is possible to prevent the microneedles 11a from coming into contact with nerves, to reduce a possibility of pain and to avoid a possibility of bleeding. When the length of the microneedles 11a is 500 µm or less, an amount of active ingredient that should enter the skin can be administered efficiently, and it becomes possible to administer the active ingredient without perforating the basement membrane, for example. In one example, the length of the microneedles 11a is selected so that they do not penetrate the stratum corneum of the skin in normal use, but it is possible that some of the microneedles 11a may penetrate the stratum corneum. In other words, the microneedles 11a stretch and thin the epidermis, making it easier for the active ingredient to penetrate into the epidermis, but it is possible that some of the active ingredient may enter the skin through holes formed in the stratum corneum.

In the example of FIG. 4, each of the microneedles 11a has a three-dimensional shape. For example, the microneedle 11a may have a conical shape or any other pyramid shape such as a square pyramid. The microneedle 11a does not have to have a conical shape, for example, the tip end portion may be flat or rounded. The flat or rounded tip end portion may be obtained by intentional machining or may be obtained naturally without such machining. When the tip end portion is flat, an area of the flat portion may be 20 to 600 µm² or 50 to 250 µm². When the tip end portion is rounded, a radius of curvature of the tip end portion may be 2 to 100 µm or 5 to 30 µm.

In one example, the microneedles 11a may or may not be precoated with an active ingredient. When the microneedles 11a are not coated with an active ingredient, the active ingredient may be applied to the skin and then the microneedle device 11 may be applied to that portion using the applicator 1A to administer the active ingredient. Alternatively, the active ingredient may be administered by applying the microneedle device 11 to the skin using the applicator 1A and then spreading the active ingredient on the application site.

The main body 2 is a substantially cylindrical structure that accommodates each component such as the switch 3. The main body 2 has a bottom portion 21, a top portion 22, a first side portion 23 (a side portion) and a second side portion 24. The bottom portion 21 is a portion of the applicator 1A that comes into contact with the skin when the applicator 1A is placed on the skin to apply the microneedle device 11 to the skin. The bottom portion 21 has a substantially circular shape. An opening 21a for inserting the microneedle device 11 into the main body 2 is formed in a substantially center of the bottom portion 21. The top portion 22 is a portion opposite to the bottom portion 21 and is thus located away from the skin when the applicator 1A is placed on the skin.

In the following description, a direction in which the bottom portion 21 and the top portion 22 face each other is referred to as a height direction D1. The height direction D1 can be said to be from the bottom portion 21 toward the top portion 22. Two directions that extend along an imaginary horizontal plane orthogonal to the height direction D1 and that are orthogonal to each other are called a lateral direction D2 and a width direction D3. Thus, the height direction D1, the lateral direction D2, and the width direction D3 are orthogonal to each other. Both the lateral direction D2 and the width direction D3 can be said to be directions from the first side portion 23 toward the center of the main body 2. Further, the side of the top portion 22 in the height direction D1 may be referred to as the upper side of the applicator 1A, and the side of the bottom portion 21 in the height direction D1 may be referred to as the lower side of the applicator 1A.

The first side portion 23 is a portion that extends in the height direction D1 and has a substantially cylindrical shape that defines an outer circumference of the main body 2. As an example, the first side portion 23 extends from the entire outer edge of the bottom portion 21 in the height direction D1 along the circumferential direction of the bottom portion 21. A top surface of the first side portion 23 may be present or function as the top portion 22. As an example, in order to improve user convenience, a height of a portion of the first side portion 23 from the bottom portion 21 (for example, a portion on the side of a fixed end 31a of an arm 31) may be lower than a height of the other portion of the first side portion 23 from the bottom portion 21.

The second side portion 24 is a portion that extends in the height direction D1 from the bottom portion 21 inside the first side portion 23. The second side portion 24 is formed substantially along an edge of the opening 21a and is connected to an inner surface of the first side portion 23. In one example, a height of the second side portion 24 from the bottom portion 21 is lower than the height of the first side portion 23 from the bottom portion 21. The height refers to a length in the height direction D1.

As an example, the main body 2 is formed to be line-symmetrical with respect to an imaginary center line CL1 parallel to the lateral direction D2 as a reference axis. In other words, each of the bottom portion 21, the top portion 22, the first side portion 23, and the second side portion 24 is formed to be line-symmetrical with respect to the center line CL1 as the reference axis.

The switch 3 is a component that is operated by the user to push the microneedle device 11 toward the skin. The switch 3 has the arm 31 and a piston 32. The arm 31 is a substantially rectangular plate-shaped portion mounted on the first side portion 23 to extend in the lateral direction D2. Therefore, the lateral direction D2 may also be referred to as an extension direction of the arm 31. The arm 31 has the fixed end 31a mounted on the first side portion 23 and a free end 31b located on the side opposite to the fixed end 31a. The arm 31 can move in an arc shape in the height direction D1 with the fixed end 31a as a fulcrum, and this movement allows the free end 31b to move in the height direction D1. The arm 31 may be formed of a member or material that generates an elastic force, and may be, for example, an elastic member such as a leaf spring. Alternatively, the arm 31 may be a non-elastic member or a flexible member. The piston 32 is a plate-shaped portion mounted on the free end 31b. The piston 32 has a piston surface 32a which is a surface facing the skin. In one example, the switch 3 is formed to be line-symmetrical with respect to the center line CL1 as a reference axis. As an example, the arm 31 extends parallel to the lateral direction D2 when seen in the width direction D3. The arm 31 may extend to be inclined with respect to the lateral direction D2 when seen in the width direction D3.

In the set state, the microneedle device 11 is located between the piston 32 and the bottom portion 21. The microneedle device 11 is disposed in the main body 2 so that the microneedle device 11 is located between the piston 32 and the skin when the bottom portion 21 comes into contact with the skin. In other words, it can be said that the piston 32, the microneedle device 11, and the bottom portion 21 (the skin) are arranged in this order in the height direction D1. The piston 32 is pushed in by the user toward the skin when the microneedle device 11 is applied to the skin. In other words, the piston 32 is pushed in by the user to the side of the bottom portion 21 in the height direction D1. At this time, the arm 31 moves in an arc shape with the fixed end 31a as a fulcrum in conjunction with the movement of the piston 32. The piston surface 32a comes into contact with the microneedle device 11 when the piston 32 is pushed in by the user toward the skin. The piston surface 32a may or may not be in contact with the microneedle device 11 in the set state.

When the applicator 1A is seen in the height direction D1, the entire switch 3 is located inside the opening 21a. Thus, the switch 3 does not come into contact with the bottom portion 21 when the piston 32 is pushed in by the user toward the skin.

When the applicator 1A is seen from the side of the top portion 22 in the height direction D1, the top portion 22 of the main body 2 does not cover at least the piston 32 and does not cover the entire switch 3, for example. The applicator 1A does not have a portion that covers the piston 32 from the side of the top portion 22 and does not have a portion that covers the entire switch 3 from the side of the top portion 22, for example. Therefore, when the applicator 1A is seen from the side of the top portion 22 in the height direction D1, the piston 32 is exposed to the outside of the applicator 1A.

The stopper 4 is a component that supports the microneedle device 11 which is disposed in the main body 2 to be located between the piston 32 and the skin. As described above, the applicator 1A includes one or more stoppers 4, for example, two stoppers 4. The two stoppers 4 may be provided to be line-symmetrical with respect to an imaginary center line (the center line CL1) of the arm 31 in the extension direction of the arm 31 as the reference axis. As an example, the two stoppers 4 are located to sandwich a vicinity of the free end 31b of the arm 31 in the width direction D3. The two stoppers 4 are provided along the outer edge of the microneedle device 11 in the set state. Each of the stoppers 4 has a columnar shape which extends in the height direction D1 from the bottom portion 21. Since a gap is formed between the entire top surface and side surface of the stopper 4 and other components of the main body 2, the stopper 4 can bend in a direction orthogonal to the height direction D1.

A protrusion 41 that protrudes toward the piston 32 when seen in the height direction D1 is provided on the stopper 4. That is, the protrusion 41 protrudes toward the center of the microneedle device 11 in the set state. The protrusion 41 overlaps the opening 21a and does not overlap the switch 3 when seen in the height direction D1. Thus, when the switch 3 moves, the protrusion 41 does not come into contact with the switch 3. The stopper 4 has a placement surface 4a on which the microneedle device 11 is disposed in the set state. The placement surface 4a is a surface facing upward and is inclined with respect to a plane orthogonal to the height direction D1. The placement surface 4a may be parallel to a plane orthogonal to the height direction D1.

The stopper 4 is provided closer to the arm 31 than an end portion 32b of the piston 32 on the center line CL1. The end portion 32b is also an end portion of the switch 3 on the center line CL1 and is located on the side opposite to the fixed end 31a. Therefore, the stopper 4 mainly supports a portion of the microneedle device 11 on the side of the arm 31 in the set state. As an example, the stopper 4 supports the peripheral edge portion 11c. The stopper 4 may support the platform surface 11d when it is not in contact with the microneedles 11a.

When the arm 31 generates an elastic force, like an elastic member such as a leaf spring, the arm 31 deforms toward the top portion 22, and thus an elastic force is generated in the arm 31. As an example, the microneedle device 11 inserted into the main body 2 through the opening 21a pushes the piston 32 upward, thereby causing the arm 31 to deform toward the top portion 22. Alternatively, the arm 31 is deformed toward the top portion 22 by such an insertion operation by the user. The switch 3, for example, the piston 32, transmits the elastic force generated in the arm 31 to the microneedle device 11 supported by the stopper 4. At this time, the switch 3 comes into contact with the microneedle device 11. The arm 31 may come into contact with the microneedle device 11, or the piston surface 32a may come into contact with the microneedle device 11. The stopper 4 supports the microneedle device 11 against the elastic force. In this way, the microneedle device 11 is supported against the elastic force by being gripped by the switch 3 and the stopper 4.

The rib 5 is a component that supports the microneedle device 11 together with the stopper 4. The rib 5 is provided at a position different from that of the stopper 4. As an example, the rib 5 is located on the imaginary center line (the center line CL1) of the arm 31 in the extension direction of the arm 31. The rib 5 is provided closer to the end portion 32b than the arm 31. Therefore, in the set state, the rib 5 mainly supports a portion of the microneedle device 11 on the side of the end portion 32b. The rib 5 has an inverted L shape that extends from the bottom portion 21 to the top portion 22 in the height direction D1 and then bends toward the center of the main body 2. A tip end surface 5a of the rib 5 faces the center of the main body 2. Since a gap is formed between the entire top surface and side surfaces of the rib 5 and other components of the main body 2, the rib 5 can bend in a direction orthogonal to the height direction D1. As described above, the applicator 1A may have a single rib 5 or two or more ribs 5. For example, the two ribs 5 may be provided to be line-symmetrical with respect to the center line CL1 as the reference axis, as in the stopper 4.

The rib 5 comes into contact with at least the side surface 11e of the microneedle device 11 which is supported by the stopper 4. As an example, the tip end surface 5a of the rib 5 comes into contact with the side surface 11e and does not come into contact with any portion of the microneedle device 11 other than the side surface 11e. The rib 5 may push the microneedle device 11 toward the stopper 4. It can be said that the stopper 4 supports the microneedle device 11 from below, whereas the rib 5 supports the microneedle device 11 from the side.

The eaves portion 6 is a component that stops the movement of the microneedle device 11 within the main body 2 so that the microneedle device 11, which is inserted into the main body 2 through the opening 21a and moves toward the top portion 22, does not pop out above the main body 2. As described above, the applicator 1A has one or more eaves portions 6, for example, two eaves portions 6. As an example, the two eaves portions 6 are provided to be line-symmetrical with respect to the imaginary center line (the center line CL1) of the arm 31 in the extension direction of the arm 31 as a reference axis. The eaves portions 6 are provided closer to the end portion 32b of the piston 32 than to the arm 31. The eaves portions 6 are located on the side opposite to the stopper 4 with the piston 32 interposed therebetween. The eaves portions 6 protrude from the second side portion 24 toward the inside of the main body 2 when seen in the height direction D1. The eaves portions 6 overlap the opening 21a and do not overlap the switch 3 when seen in the height direction D1. Thus, when the switch 3 moves, the eaves portions 6 do not come into contact with the switch 3.

The eaves portions 6 are provided closer to the top portion 22 than the placement surface 4a of the stopper 4 in the height direction D1. In other words, the eaves portions 6 are located above the stopper 4. A distance between lower ends 6a of the eaves portions 6 and an upper end 4b of the placement surface 4a in the height direction D1 is greater than a thickness of the peripheral edge portion 11c of the microneedle device 11. From the viewpoint of holding the microneedle device 11 more stably, the distance between the lower ends 6a of the eaves portions 6 and the upper end 4b of the placement surface 4a may be the same as or smaller than the thickness of the peripheral edge portion 11c of the microneedle device 11. The lower ends 6a are ends of the eaves portions 6 that are located on the side of the bottom portion 21 in the height direction D1. The upper end 4b is an end of the placement surface 4a that is located on the side of the top portion 22 in the height direction D1.

The eaves portions 6 are provided to cover at least a part of the peripheral edge portion 11c of the microneedle device 11 when the microneedle device 11 supported by the stopper 4 is seen from the side of the top portion 22 in the height direction D1. As an example, the eaves portions 6 cover a part of the peripheral edge portion 11c. As an example, the eaves portions 6 are provided partially along the peripheral edge portion 11c so that other portions of the peripheral edge portion 11c are visible when the microneedle device 11 is seen from the side of the top portion 22 in the height direction D1. In the applicator 1A, the eaves portions 6 are provided at two locations along the peripheral edge portion 11c.

The recovery mechanism 7 is a component that holds the microneedle device 11, at least when the microneedle device 11 is applied to the skin by an operation of the switch 3 by the user. The recovery mechanism 7 has two gripping claws 71 that grip the microneedle device 11. As an example, the gripping claws 71 grip the microneedle device 11 before the microneedle device 11 reaches the skin in response to the piston 32 moving toward the skin. Each of the two gripping claws 71 is provided on the piston 32 to be located outside the piston surface 32a, and is located closer to the bottom portion 21 than the piston surface 32a in the height direction D1. As an example, the two gripping claws 71 are located on both sides of the piston surface 32a in the width direction D3 and grip the microneedle device 11 from both sides thereof. As an example, the two gripping claws 71 are located on an imaginary center line CL2 parallel to the width direction D3 of the microneedle device 11 in the set state. In this case, the gripping claws 71 can stably grip the microneedle device 11.

Next, a series of operations until the microneedle device 11 is applied to the skin will be described with reference to FIG. 4.

In the set state shown as scene S101, the microneedle device 11 is supported from below by the stopper 4 and from the side by the rib 5. At this time, the piston 32 of the switch 3 is located above the microneedle device 11 and the gripping claws 71 do not grip the microneedle device 11.

In scene S102, the user pushes the switch 3 toward the skin, and the gripping claws 71 come into contact with the microneedle device 11. Since the stopper 4 continues to support the microneedle device 11, the two gripping claws 71 in contact with the microneedle device 11 open outward, then pass along the sides of the microneedle device 11 and reach below the peripheral edge portion 11c of the microneedle device 11. As a result, the two gripping claws 71 grip the microneedle device 11. As an example, the two gripping claws 71 grip the peripheral edge portion 11c and do not grip the platform surface 11d. When the gripping claws 71 grip the microneedle device 11, the substantially entire surface of the piston surface 32a is in contact with the microneedle device 11. The gripping claws 71 may grip the platform surface 11d as long as they do not come into contact with the microneedles 11a.

In scene S103, as the user pushes the switch 3 further in, the microneedle device 11 pushed by the piston 32 passes over the protrusion 41, moves downward, and reaches the skin. At this time, since the rib 5 bends outward in the direction orthogonal to the height direction D1, the microneedle device 11 moves smoothly downward. Thus, the microneedles 11a come into contact with or pierce the skin. In this way, the microneedle device 11 is applied to the skin.

The user then moves the applicator 1A away from the skin. The gripping claws 71 grip the microneedle device 11 even after the microneedle device 11 has been applied to the skin. Therefore, when the user moves the applicator 1A away from the skin, the microneedle device 11 gripped by the gripping claws 71 is also moved away from the skin together with the applicator 1A.

As described above, the gripping claws 71 grip the microneedle device 11 before the microneedle device 11 passes over the protrusion 41. As another example, the gripping claws 71 may grip the microneedle device 11 at the time the microneedle device 11 passes over the protrusion 41 or after the microneedle device 11 passes over the protrusion 41. For example, the gripping claws 71 may grip the microneedle device 11 at the time the microneedle device 11 reaches the skin. Alternatively, the gripping claws 71 may grip the microneedle device 11 as shown in scene S102, even before the user pushes the switch 3 toward the skin.

### [Application Kit According to First Example]

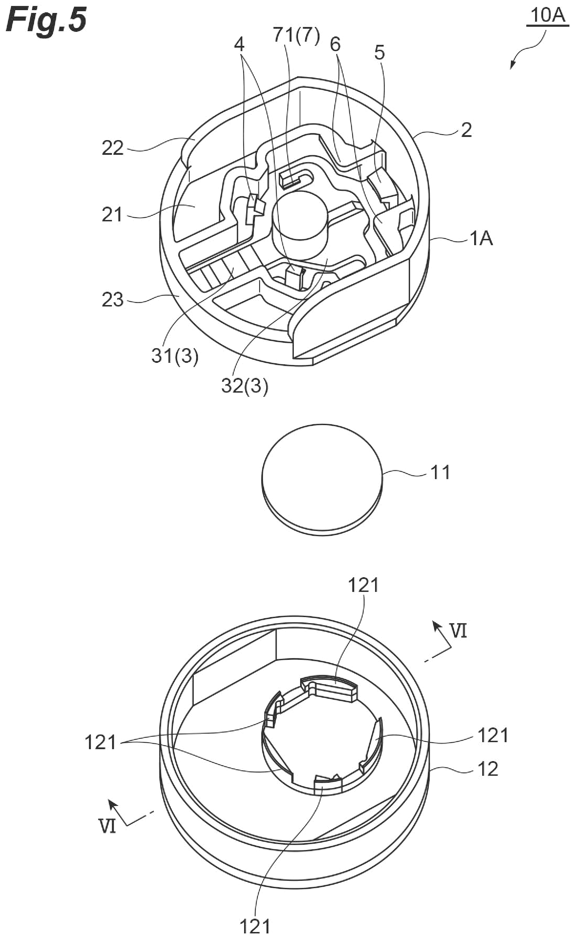

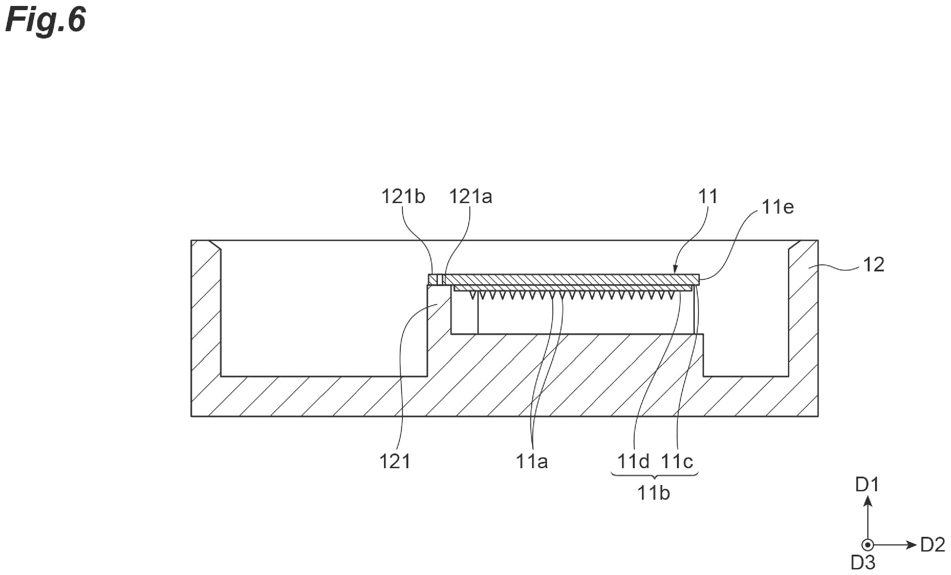

With reference to FIGS. 5 and 6, a configuration of an application kit 10A according to the first example will be described. FIG. 5 is a perspective view of the application kit 10A seen from above. FIG. 6 is a cross-sectional view along line VI-VI shown in FIG. 5. FIG. 6 shows a state in which the microneedle device 11 is disposed on a stage 12.

The stage 12 is an article for arranging the microneedle device 11 in the applicator 1A. The stage 12 has a columnar portion 121 that extends in the height direction D1 and supports the microneedle device 11. The columnar portion 121 is provided partially along the peripheral edge portion 11c of the microneedle device 11. The columnar portion 121 has a top surface 121a that supports the microneedle device 11 and a protrusion 121b that protrudes from the top surface 121a in the height direction D1. The top surface 121a is a surface that faces the microneedle device 11. As an example, the top surface 121a supports the peripheral edge portion 11c and is not in contact with the platform surface 11d. The protrusion 121b is located outside the microneedle device 11 when the microneedle device 11 supported by the columnar portion 121 is seen from the side of the microneedle device 11 in the height direction D1. The protrusion 121b may or may not be in contact with the side surface 11e of the microneedle device 11.

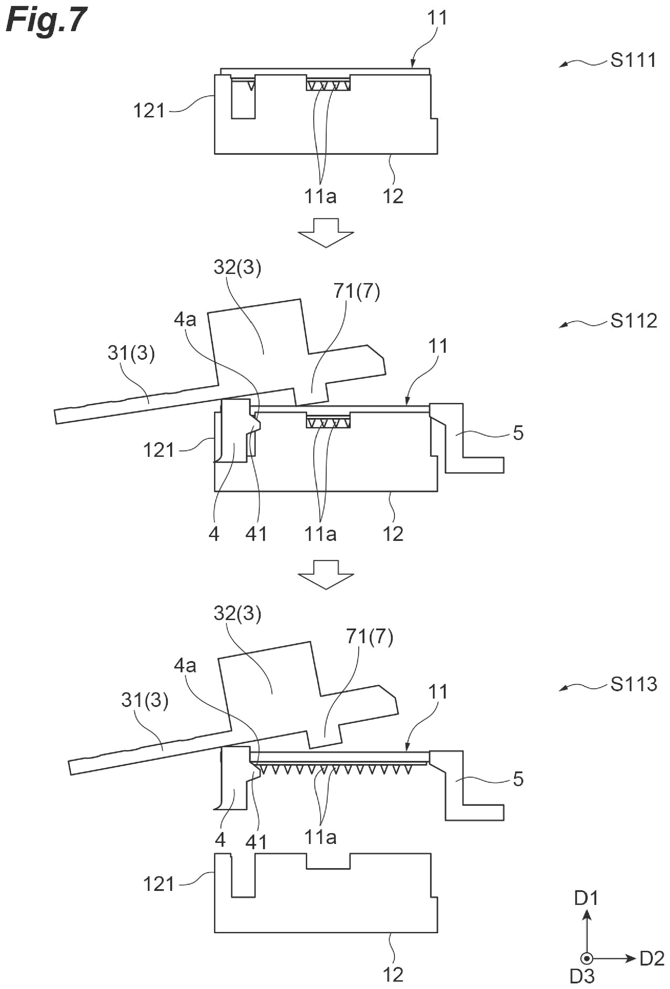

Referring to FIG. 7, a process of arranging the microneedle device 11 disposed on the stage 12 in the applicator 1A will be described. The main body 2 is omitted in FIG. 7.

In scene S111, the user aligns the position of the peripheral edge portion 11c of the microneedle device 11 with the top surface 121a of the columnar portion 121 and places the microneedle device 11 on the columnar portion 121. By this operation, the microneedle device 11 is disposed on the stage 12.

In scene S112, the user aligns the position of the applicator 1A with the stage 12 and places the applicator 1A into the stage 12 from above the stage 12. By this operation, the columnar portion 121 that supports the microneedle device 11 is inserted into the main body 2 toward the piston 32. By this insertion, the piston 32 is pushed by the microneedle device 11 inside the main body 2 and moves upward, and the microneedle device 11 enters between the piston 32 and the stopper 4 and is supported by the stopper 4. As an example, the microneedle device 11 passes over the protrusion 41 of the stopper 4, whereby the microneedle device 11 is disposed on the placement surface 4a. At this time, the rib 5 supports the microneedle device 11 from the side. When the microneedle device 11 passes over the protrusion 41, the stopper 4 and the rib 5 bend outward in the direction orthogonal to the height direction D1, and thus a positional relationship between the microneedle device 11 and the columnar portion 121 does not change. Since the positional relationship does not change, the microneedles 11a do not come into contact with the columnar portion 121, and thus it is possible to curb the microneedle 11a breaking. When the columnar portion 121 that supports the microneedle device 11 is inserted into the main body 2 toward the piston 32, the piston 32 may be pushed by the columnar portion 121 and move upward.

In scene S113, the user moves the applicator 1A, on which the microneedle device 11 is set, above the stage 12 and moves the applicator 1A away from the stage 12. This applicator 1A is in the set state as shown in scene S101 described above. As described with reference to FIG. 4, the user operates the applicator 1A on the skin. This operation operates the applicator 1A to apply the microneedle device 11 to the skin as shown in scenes S102 and S103 described above.

As described above, in the application kit 10A, the gripping claws 71 do not grip the microneedle device 11 from the time the microneedle device 11 is inserted into the main body 2 toward the piston 32 until it is supported by the stopper 4.

### [Applicator According to Second Example]

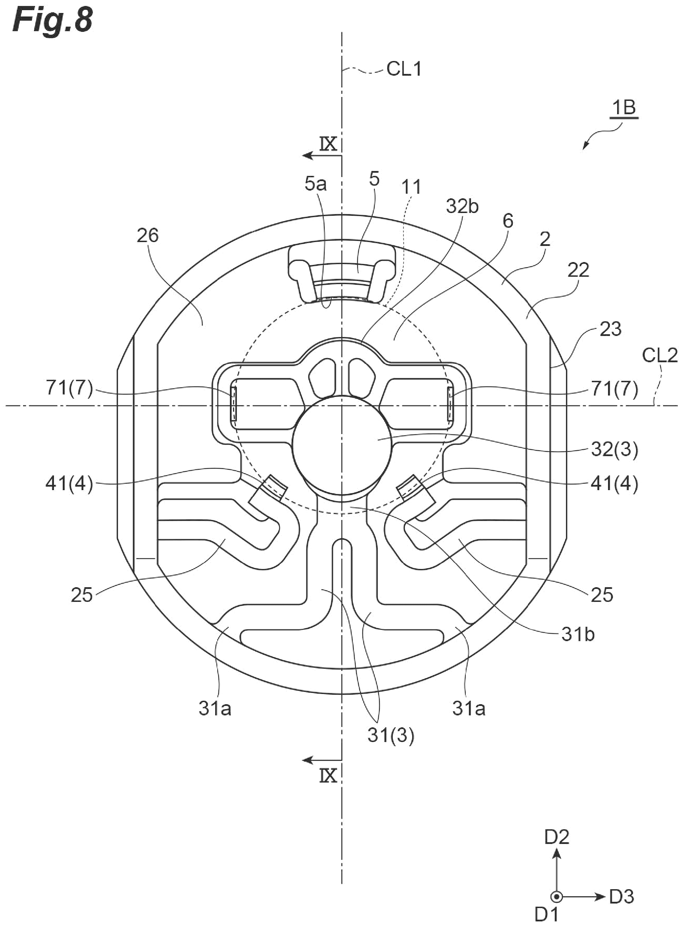

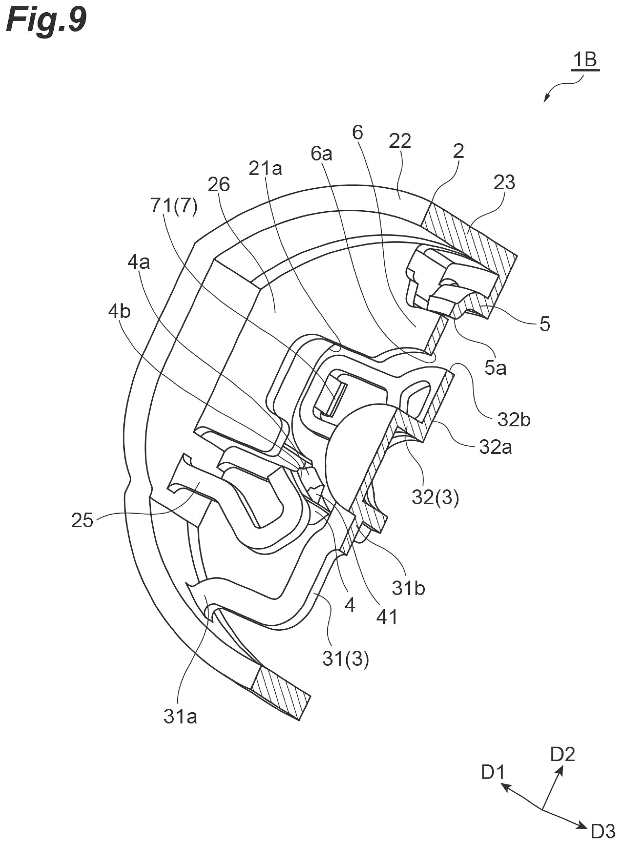

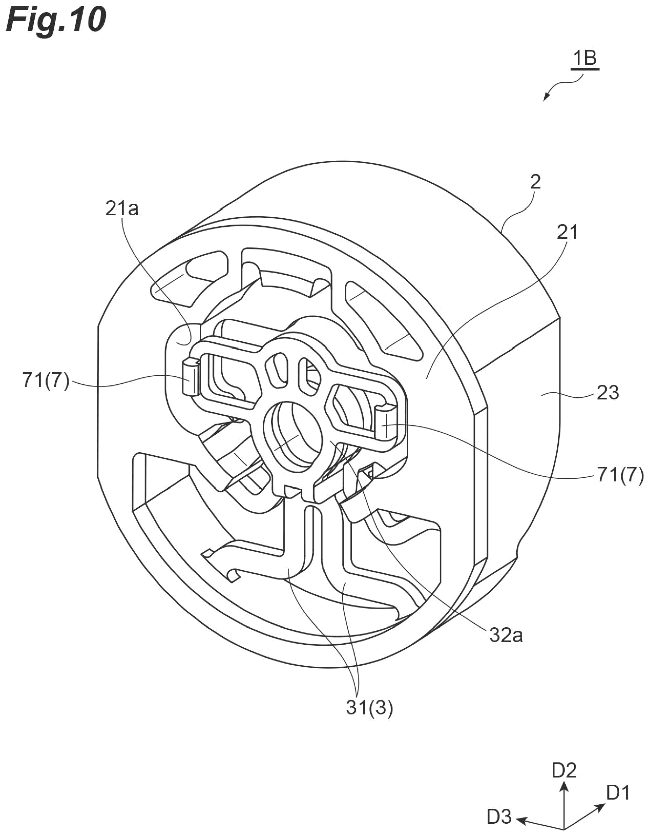

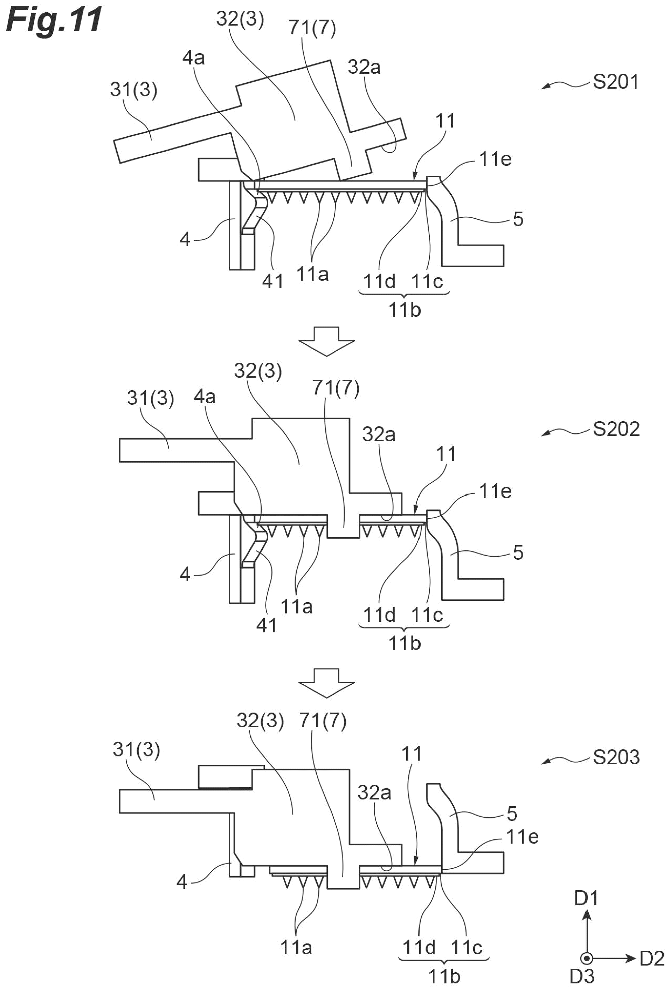

With reference to FIGS. 8 to 11, a structure of an applicator 1B according to a second example will be described. FIG. 8 is a plan view of the applicator 1B seen from above. FIG. 9 is a perspective cross-sectional view along line IX-IX shown in FIG. 8. FIG. 10 is a perspective view of the applicator 1B seen from below. FIG. 11 is a diagram showing a process from a state in which the microneedle device 11 is set in the applicator 1B until the microneedle device 11 is applied to the skin. FIGS. 8 to 10 show the applicator 1B in the natural state.

The applicator 1B includes a main body 2, a switch 3, at least one stopper 4, at least one rib 5, at least one eaves portion 6, and a recovery mechanism 7. A material of the applicator 1B is the same as that of the applicator 1A, for example. A configuration of the microneedle device 11 set in the applicator 1B is the same as that of the microneedle device 11 set in the applicator 1A, for example.

The main body 2 is a substantially cylindrical structure that accommodates each component such as the switch 3. The main body 2 has a bottom portion 21, a top portion 22, a first side portion 23 (a side portion) and a movable portion 25. The bottom portion 21 is a portion that comes into contact with the skin when the applicator 1B is placed on the skin to apply the microneedle device 11 to the skin. The bottom portion 21 has a substantially circular shape. An opening 21a for inserting the microneedle device 11 into the main body 2 is formed over most of the bottom portion 21. The top portion 22 is a portion opposite to the bottom portion 21 and is thus located away from the skin when the applicator 1B is placed on the skin.

Definitions of height direction D1, lateral direction D2, and width direction D3 used in the following description are the same as the definitions of the height direction D1, the lateral direction D2, and the width direction D3 used in the description of the first example. Further, the side of the top portion 22 in the height direction D1 may be referred to as the upper side of the applicator 1B, and the side of the bottom portion 21 in the height direction D1 may be referred to as the lower side of the applicator 1B.

The first side portion 23 is a portion that extends in the height direction D1 and has a substantially cylindrical shape that defines an outer circumference of the main body 2. As an example, the first side portion 23 extends from the entire outer edge of the bottom portion 21 in the height direction D1 along the circumferential direction of the bottom portion 21. A top surface of the first side portion 23 may be present or function as the top portion 22. As an example, in order to improve user convenience, a height of a portion of the first side portion 23 from the bottom portion 21 (for example, a portion on the side of a fixed end 31a of an arm 31) may be lower than a height of the other portion of the first side portion 23 from the bottom portion 21.

The movable portion 25 is a bar-shaped portion that extends from an inner surface of the first side portion 23, while bending toward the stopper 4. Since a gap is formed between the entire surface of the movable portion 25 and the other components of the main body 2, the movable portion 25 can bend in a direction orthogonal to the height direction D1.

As an example, the main body 2 is formed to be line-symmetrical with respect to the imaginary center line CL1 parallel to the lateral direction D2 as a reference axis. In other words, each of the bottom portion 21, the top portion 22, the first side portion 23, and the movable portion 25 is formed to be line-symmetrical with respect to the center line CL1 as the reference axis.

The switch 3 is a component that is operated by the user to push the microneedle device 11 toward the skin. The switch 3 has the arm 31 and a piston 32. The arm 31 is mounted on the first side portion 23 to extend in the lateral direction D2. Therefore, the lateral direction D2 may also be referred to as an extension direction of the arm 31. The majority of the arm 31 is in the shape of a bifurcated bar, and the bifurcated bar portion is mounted on the first side portion 23. The arm 31 has a fixed end 31a mounted on the first side portion 23, and a free end 31b located on the side opposite to the fixed end 31a. The arm 31 can move in an arc shape in the height direction D1 with the fixed end 31a as a fulcrum, and this movement allows the free end 31b to move in the height direction D1. The arm 31 may be formed of a member or material that generates an elastic force, and may be, for example, an elastic member such as a leaf spring. Alternatively, the arm 31 may be a non-elastic member or a flexible member. The piston 32 is mounted on the free end 31b and is a plate-shaped portion having an opening in most of its area. The piston 32 has a piston surface 32a which is a surface facing the skin. In one example, the switch 3 is formed to be line-symmetrical with respect to the center line CL1. As an example, the arm 31 extends parallel to the lateral direction D2 when seen in the width direction D3. The arm 31 may extend to be inclined with respect to the lateral direction D2 when seen in the width direction D3.

In the set state, the microneedle device 11 is located between the piston 32 and the bottom portion 21. The microneedle device 11 is disposed in the main body 2 so that the microneedle device 11 is located between the piston 32 and the skin when the bottom portion 21 comes into contact with the skin. In other words, it can be said that the piston 32, the microneedle device 11, and the bottom portion 21 (the skin) are arranged in this order in the height direction D1. The piston 32 is pushed in by the user toward the skin when the microneedle device 11 is applied to the skin. In other words, the piston 32 is pushed in by the user to the side of the bottom portion 21 in the height direction D1. At this time, the arm 31 moves in an arc shape with the fixed end 31a as a fulcrum in conjunction with the movement of the piston 32. The piston surface 32a comes into contact with the microneedle device 11 when it is pushed in by the user toward the skin. The piston surface 32a may or may not be in contact with the microneedle device 11 in the set state.

When the applicator 1B is seen in the height direction D1, the entire switch 3 is located inside the opening 21a. Thus, the switch 3 does not come into contact with the bottom portion 21 when the piston 32 is pushed in by the user toward the skin.

When the applicator 1B is seen from the side of the top portion 22 in the height direction D1, the top portion 22 of the main body 2 does not cover at least the piston 32 and does not cover the entire switch 3, for example. The applicator 1B does not have a portion that covers the piston 32 from the side of the top portion 22 and does not have a portion that covers the entire switch 3 from the side of the top portion 22, for example. Therefore, when the applicator 1B is seen from the side of the top portion 22 in the height direction D1, the piston 32 is exposed to the outside of the applicator 1B.

The stopper 4 is a component that supports the microneedle device 11 which is disposed in the main body 2 to be located between the piston 32 and the skin. As described above, the applicator 1B includes one or more stoppers 4, for example, two stoppers 4. The two stoppers 4 may be provided to be line-symmetrical with respect to the imaginary center line (the center line CL1) of the arm 31 in the extension direction of the arm 31 as a reference axis. As an example, the two stoppers 4 are located to sandwich a vicinity of the free end 31b of the arm 31 in the width direction D3. The two stoppers 4 are provided along the outer edge of the microneedle device 11 in the set state. Each of the stoppers 4 has a columnar shape which extends in the height direction D1 from the bottom portion 21. Each of the stoppers 4 is connected to the movable portion 25 that can bend in the direction orthogonal to the height direction D1. For other portions of the top surface and side surfaces of the stopper 4, except for a connection portion with the movable portion 25, a gap is formed between the stopper 4 and other components of the main body 2. Thus, the stopper 4 can bend in the direction orthogonal to the height direction D1.

A protrusion 41 that protrudes toward the piston 32 when seen in the height direction D1 is provided on the stopper 4. That is, the protrusion 41 protrudes toward the center of the microneedle device 11 in the set state. The protrusion 41 overlaps the opening 21a and does not overlap the switch 3 when seen in the height direction D1. Thus, when the switch 3 moves, the protrusion 41 does not come into contact with the switch 3. The stopper 4 has a placement surface 4a on which the microneedle device 11 is disposed in the set state. The placement surface 4a is a surface facing upward and is inclined with respect to a plane orthogonal to the height direction D1. The placement surface 4a may be parallel to a plane orthogonal to the height direction D1.

The stopper 4 is provided closer to the arm 31 than an end portion 32b of the piston 32 on the center line CL1. The end portion 32b is also an end portion of the switch 3 on the center line CL1 and is located on the side opposite to the fixed end 31a. Therefore, the stopper 4 mainly supports a portion of the microneedle device 11 on the side of the arm 31 in the set state. As an example, the stopper 4 supports the peripheral edge portion 11c. The stopper 4 may support the platform surface 11d when it does not come into contact with the microneedles 11a.

When the arm 31 generates an elastic force, like an elastic member such as a leaf spring, the arm 31 deforms toward the top portion 22, and thus an elastic force is generated in the arm 31. As an example, the microneedle device 11 inserted into the main body 2 through the opening 21a pushes the piston 32 upward, thereby causing the arm 31 to deform toward the top portion 22. Alternatively, the arm 31 is deformed toward the top portion 22 by such an insertion operation by the user. The switch 3, for example, the piston 32, transmits the elastic force generated in the arm 31 to the microneedle device 11 supported by the stopper 4. At this time, the switch 3 comes into contact with the microneedle device 11. The arm 31 may come into contact with the microneedle device 11, or the piston surface 32a may come into contact with the microneedle device 11. The stopper 4 supports the microneedle device 11 against the elastic force. In this way, the microneedle device 11 is gripped by the switch 3 and the stopper 4 and thus supported against the elastic force.

The rib 5 is a component that supports the microneedle device 11 together with the stopper 4. The rib 5 is provided at a position different from that of the stopper 4. As an example, the rib 5 is located on the imaginary center line (the center line CL1) of the arm 31 in the extension direction of the arm 31. The rib 5 is provided closer to the end portion 32b than the arm 31. Therefore, in the set state, the rib 5 mainly supports a portion of the microneedle device 11 on the side of the end portion 32b. The rib 5 has an inverted L shape that extends from the bottom portion 21 to the top portion 22 in the height direction D1 and then bends toward the center of the main body 2. A tip end surface 5a of the rib 5 faces the center of the main body 2. Since a gap is formed between the entire top surface and side surfaces of the rib 5 and other components of the main body 2, the rib 5 can bend in a direction orthogonal to the height direction D1. As described above, the applicator 1B may have a single rib 5 or two or more ribs 5. For example, the two ribs 5 may be provided to be line-symmetrical with respect to the center line CL1 as the reference axis, as in the stopper 4.

The rib 5 comes into contact with at least the side surface 11e of the microneedle device 11 which is supported by the stopper 4. As an example, the tip end surface 5a of the rib 5 comes into contact with the side surface 11e and does not come into contact with any portion of the microneedle device 11 other than the side surface 11e. The rib 5 may push the microneedle device 11 toward the stopper 4. It can be said that the stopper 4 supports the microneedle device 11 from below, whereas the rib 5 supports the microneedle device 11 from the side.

The eaves portion 6 is a component that stops the movement of the microneedle device 11 within the main body 2 so that the microneedle device 11, which is inserted into the main body 2 through the opening 21a and moves toward the top portion 22, does not pop out above the main body 2. As described above, the applicator 1B has one or more eaves portions 6, for example, one eaves portion 6. As an example, the eaves portion 6 is provided to be line-symmetrical with respect to the imaginary center line (the center line CL1) of the arm 31 in the extension direction of the arm 31 as a reference axis. The eaves portion 6 is provided closer to the end portion 32b of the piston 32 than to the arm 31. The eaves portion 6 is located on the side opposite to the stopper 4 with the piston 32 interposed therebetween. In the applicator 1B, the eaves portion 6 is a part of a wall portion 26 that extends from a substantially upper half (a substantially half located at the upper portion of FIG. 8) of the first side portion 23 toward the inside of the main body 2 when seen in the height direction D1. The eaves portion 6 overlaps the opening 21a and does not overlap the switch 3 when seen in the height direction D1. Thus, when the switch 3 moves, the eaves portion 6 does not come into contact with the switch 3.

The eaves portion 6 is provided closer to the top portion 22 than the placement surface 4a of the stopper 4 in the height direction D1. In other words, the eaves portion 6 is located above the stopper 4. A distance between a lower end 6a of the eaves portion 6 and an upper end 4b of the placement surface 4a in the height direction D1 is greater than a thickness of the peripheral edge portion 11c of the microneedle device 11. From the viewpoint of holding the microneedle device 11 more stably, the distance between the lower end 6a of the eaves portion 6 and the upper end 4b of the placement surface 4a may be the same as or smaller than the thickness of the peripheral edge portion 11c of the microneedle device 11. The lower end 6a is an end of the eaves portion 6 that is located on the side of the bottom portion 21 in the height direction D1. The upper end 4b is an end of the placement surface 4a that is located on the side of the top portion 22 in the height direction D1.

The eaves portion 6 is provided to cover at least a part of the peripheral edge portion 11c of the microneedle device 11 when the microneedle device 11 supported by the stopper 4 is seen from the side of the top portion 22 in the height direction D1. As an example, the eaves portion 6 covers a part of the peripheral edge portion 11c. As an example, the eaves portion 6 is provided partially along the peripheral edge portion 11c so that other portions of the peripheral edge portion 11c are visible when the microneedle device 11 is seen from the side of the top portion 22 in the height direction D1. In the applicator 1B, the eaves portion 6 is provided at one location along the peripheral edge portion 11c.

The recovery mechanism 7 is a component that holds the microneedle device 11, at least when the microneedle device 11 is applied to the skin by an operation of the switch 3 by the user. The recovery mechanism 7 has two gripping claws 71 that grip the microneedle device 11. As an example, the gripping claws 71 grip the microneedle device 11 before the microneedle device 11 reaches the skin in response to the piston 32 moving toward the skin. Each of the two gripping claws 71 is provided on the piston 32 to be located outside of the piston surface 32a, and is located closer to the bottom portion 21 than the piston surface 32a in the height direction D1. As an example, the two gripping claws 71 are located on both sides of the piston surface 32a in the width direction D3 and grip the microneedle device 11 from both sides thereof. As an example, the two gripping claws 71 are located on an imaginary center line CL2 parallel to the width direction D3 of the microneedle device 11 in the set state. In this case, the gripping claws 71 can stably grip the microneedle device 11.

Next, a series of operations until the microneedle device 11 is applied to the skin will be described with reference to FIG. 11.

In the set state shown as scene S201, the microneedle device 11 is supported from below by the stopper 4 and from the side by the rib 5. At this time, the piston 32 of the switch 3 is located above the microneedle device 11 and the gripping claws 71 do not grip the microneedle device 11.

In scene S202, the user pushes the switch 3 toward the skin, and the gripping claws 71 come into contact with the microneedle device 11. Since the stopper 4 continues to support the microneedle device 11, the two gripping claws 71 in contact with the microneedle device 11 open outward, then pass along the sides of the microneedle device 11 and reach below the peripheral edge portion 11c of the microneedle device 11. As a result, the two gripping claws 71 grip the microneedle device 11. As an example, the two gripping claws 71 grip the peripheral edge portion 11c and do not grip the platform surface 11d. When the gripping claws 71 grip the microneedle device 11, the substantially entire surface of the piston surface 32a is in contact with the microneedle device 11. The gripping claws 71 may grip the platform surface 11d when they do not come into contact with the microneedles 11a.

In scene S203, as the user pushes the switch 3 further in, the microneedle device 11 pushed by the piston 32 passes over the protrusion 41, moves downward, and reaches the skin. At this time, since the rib 5 bends outward in the direction orthogonal to the height direction D1, the microneedle device 11 moves smoothly downward. Thus, the microneedles 11a come into contact with or pierce the skin. In this way, the microneedle device 11 is applied to the skin.

The user then moves the applicator 1B away from the skin. The gripping claws 71 grip the microneedle device 11 even after the microneedle device 11 has been applied to the skin. Therefore, when the user moves the applicator 1B away from the skin, the microneedle device 11 gripped by the gripping claws 71 is also moved away from the skin together with the applicator 1B.

As described above, the gripping claws 71 grip the microneedle device 11 before the microneedle device 11 passes over the protrusion 41. As another example, the gripping claws 71 may grip the microneedle device 11 at the time the microneedle device 11 passes over the protrusion 41 or after the microneedle device 11 passes over the protrusion 41. For example, the gripping claws 71 may grip the microneedle device 11 at the time the microneedle device 11 reaches the skin. Alternatively, the gripping claws 71 may grip microneedle device 11 as shown in scene S202, even before the user pushes the switch 3 toward the skin.

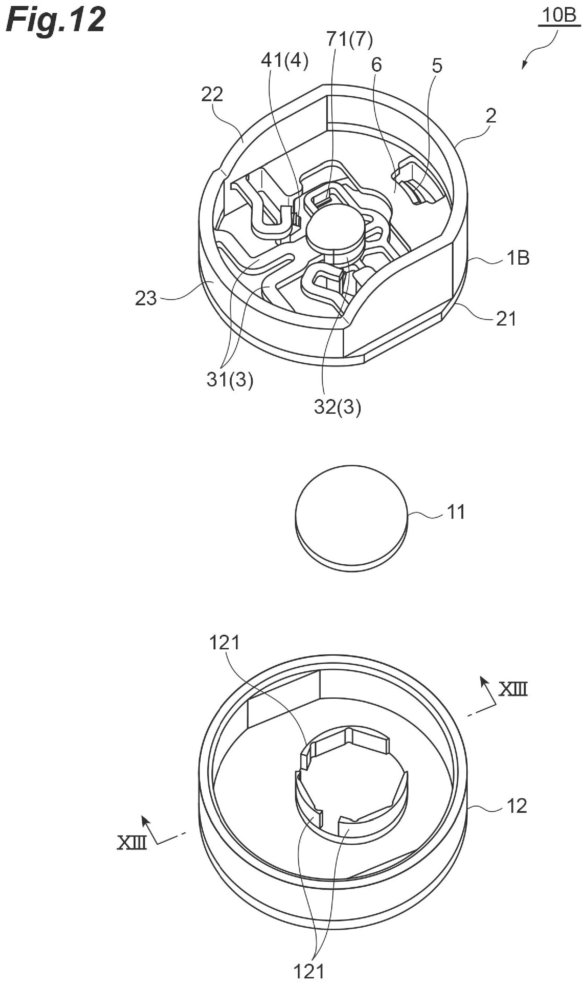

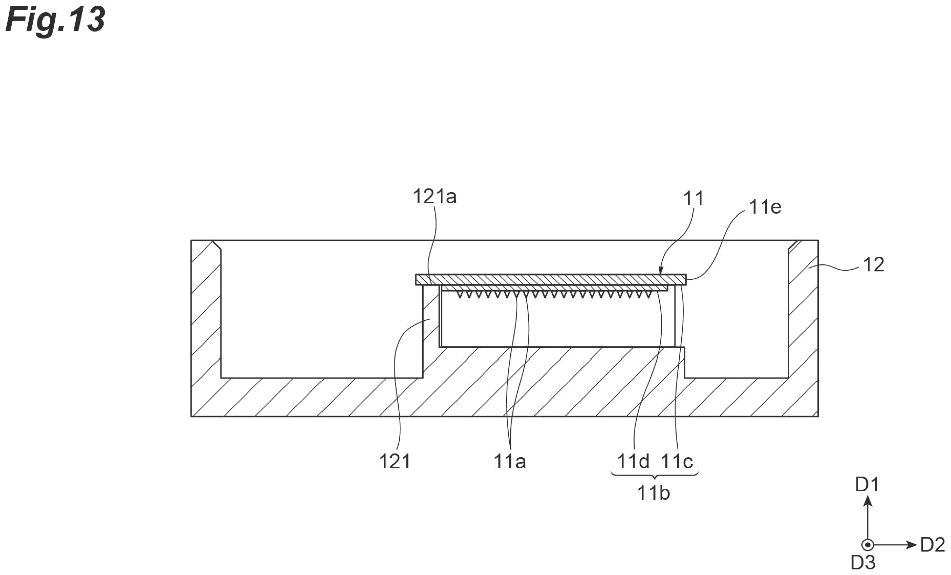

[Application Kit According to Second Example] With reference to FIGS. 12 and 13, a configuration of an application kit 10B according to the second example will be described. FIG. 12 is a perspective view of the application kit 10B seen from above. FIG. 13 is a cross-sectional view along line XIII-XIII shown in FIG. 12. FIG. 13 shows a state in which the microneedle device 11 is disposed on a stage 12.

The stage 12 is an article for arranging the microneedle device 11 in the applicator 1B. The stage 12 has a columnar portion 121 that extends in the height direction D1 to support the microneedle device 11. The columnar portion 121 is provided partially along the peripheral edge portion 11c of the microneedle device 11. The columnar portion 121 has a top surface 121a that supports the microneedle device 11. The top surface 121a is a surface that faces the microneedle device 11. As an example, the top surface 121a supports the peripheral edge portion 11c and does not come into contact with the platform surface 11d.

In the application kit 10B, a process until the microneedle device 11 disposed on the stage 12 is disposed into the applicator 1B is the same as the process in the application kit 10A described with reference to FIG. 7.

### [Applicator According to Third Example]

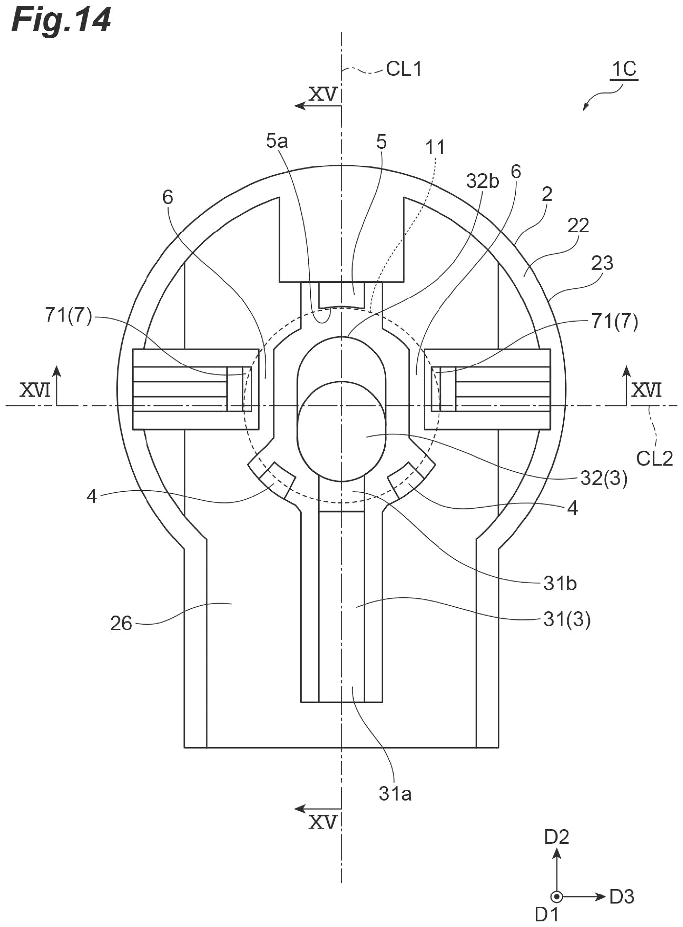

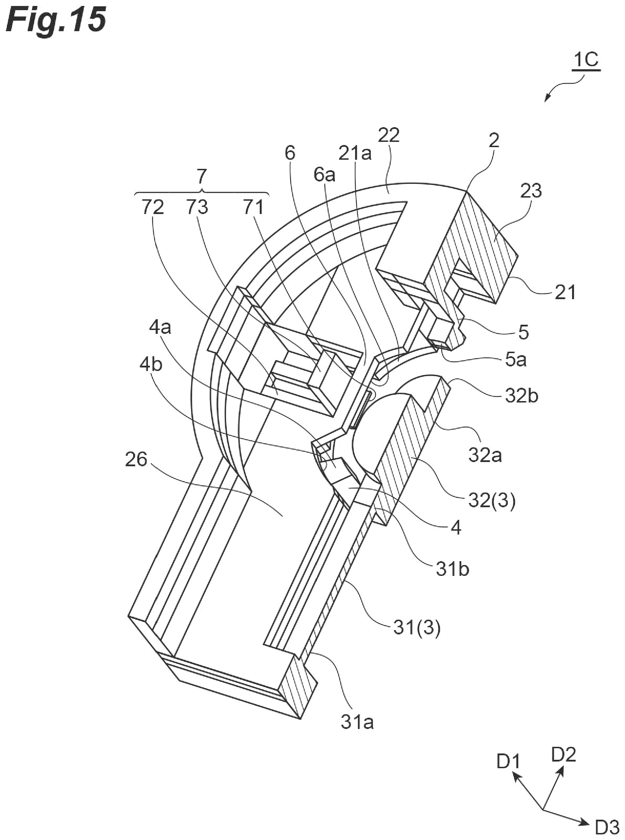

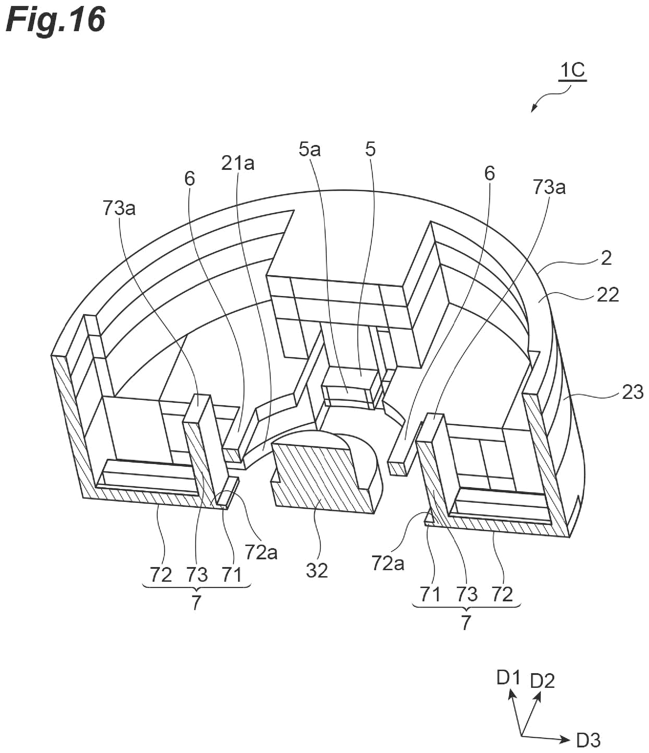

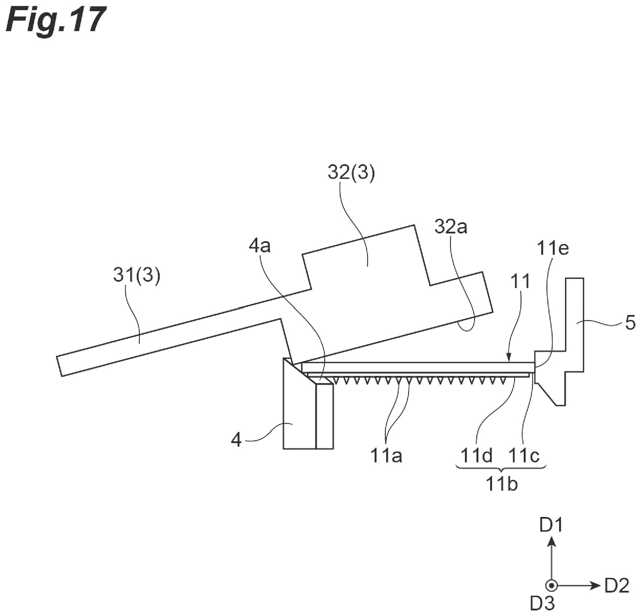

Referring to FIGS. 14 to 17, a structure of an applicator 1C according to the third example will be described. FIG. 14 is a plan view of the applicator 1C seen from above. FIG. 15 is a perspective cross-sectional view along line XV-XV shown in FIG. 14. FIG. 16 is a perspective cross-sectional view along line XVI-XVI shown in FIG. 14. FIG. 17 is a diagram showing a state in which the microneedle device 11 is set in the applicator 1C. FIGS. 14 to 16 show the applicator 1C in the natural state.

The applicator 1C includes a main body 2, a switch 3, at least one stopper 4, at least one rib 5, at least one eaves portion 6 (a blocking portion), and a recovery mechanism 7. A material of the applicator 1C is the same as that of the applicator 1A, for example. A configuration of the microneedle device 11 set in the applicator 1C is the same as that of the microneedle device 11 set in the applicator 1A.

The main body 2 is a structure that accommodates each component, such as the switch 3, and is formed by a combination of a substantially cylindrical portion and a substantially rectangular parallelepiped portion. The main body 2 has a bottom portion 21, a top portion 22, a first side portion 23 (a side portion) and a wall portion 26. The bottom portion 21 is a portion that comes into contact with the skin when the applicator 1C is placed on the skin to apply the microneedle device 11 to the skin. The bottom portion 21 is formed by a combination of a substantially circular portion and a substantially rectangular portion. An opening 21a for inserting the microneedle device 11 into the main body 2 is formed in a substantially center of the bottom portion 21. The top portion 22 is a portion opposite to the bottom portion 21 and is thus located away from that skin when the applicator 1C is placed on the skin.

Definitions of height direction D1, lateral direction D2, and width direction D3 used in the following description are the same as the definitions of the height direction D1, the lateral direction D2, and the width direction D3 used in the description of the first example. Further, the side of the top portion 22 in the height direction D1 may be referred to as the upper side of the applicator 1C, and the side of the bottom portion 21 in the height direction D1 may be referred to as the lower side of the applicator 1C.

The first side portion 23 extends in the height direction D1 and defines an outer circumference of the main body 2. As an example, the first side portion 23 extends in the circumferential direction of the bottom portion 21 and in the height direction D1 from the entire outer edge of the bottom portion 21. A top surface of the first side portion 23 may be present or function as the top portion 22. As an example, in order to improve user convenience, a height of a portion of the first side portion 23 from the bottom portion 21 (for example, a portion on the side of a fixed end 31a of an arm 31) may be lower than a height of the other portion of the first side portion 23 from the bottom portion 21.

The wall portion 26 extends from the entire inner surface of the first side portion 23 toward the inside of the main body 2. In the applicator 1C, the wall portion 26 is integrally formed with the bottom portion 21. As an example, the wall portion 26 is a thicker portion in the height direction D1 than the bottom portion 21.

As an example, the main body 2 is formed to be line-symmetrical with respect to the imaginary center line CL1 parallel to the lateral direction D2 as the reference axis. In other words, each of the bottom portion 21, the top portion 22, the first side portion 23, and the wall portion 26 is formed to be line-symmetrical with respect to the center line CL1 as a reference axis.

The switch 3 is a component that is operated by the user to push the microneedle device 11 toward the skin. The switch 3 has an arm 31 and a piston 32. The arm 31 is a rectangular plate-shaped portion mounted on the first side portion 23 to extend in the lateral direction D2. Therefore, the lateral direction D2 may also be referred to as an extension direction of the arm 31. The arm 31 has a fixed end 31a mounted on the first side portion 23, and a free end 31b located on the side opposite to the fixed end 31a. The arm 31 can move in an arc shape in the height direction D1 with the fixed end 31a as a fulcrum, and this movement allows the free end 31b to move in the height direction D1. The arm 31 may be formed of a member or material that generates an elastic force, and may be, for example, an elastic member such as a leaf spring. Alternatively, the arm 31 may be a non-elastic member or a flexible member. The piston 32 is a plate-shaped portion mounted on the free end 31b. The piston 32 has a piston surface 32a which is a surface facing the skin. In one example, the switch 3 is formed to be line-symmetrical with respect to the center line CL1. As an example, the arm 31 extends parallel to the lateral direction D2 when seen in the width direction D3. The arm 31 may extend to be inclined with respect to the lateral direction D2 when seen in the width direction D3.

In the set state, the microneedle device 11 is located between the piston 32 and the bottom portion 21. The microneedle device 11 is disposed in the main body 2 so that the microneedle device 11 is located between the piston 32 and the skin when the bottom portion 21 comes into contact with the skin. In other words, it can be said that the piston 32, the microneedle device 11, and the bottom portion 21 (the skin) are arranged in this order in the height direction D1. The piston 32 is pushed in by the user toward the skin when the microneedle device 11 is applied to the skin. In other words, the piston 32 is pushed in by the user to the side of the bottom portion 21 in the height direction D1. At this time, the arm 31 moves in an arc shape with the fixed end 31a as a fulcrum in conjunction with the movement of the piston 32. The piston surface 32a comes into contact with the microneedle device 11 when it is pushed in by the user toward the skin. The piston surface 32a may or may not be in contact with the microneedle device 11 in the set state.

When the applicator 1C is seen in the height direction D1, the entire switch 3 is located inside opening 21a. Thus, the switch 3 does not come into contact with bottom portion 21 when the piston 32 is pushed in by the user toward the skin.

When the applicator 1C is seen from the side of top portion 22 in the height direction D1, the top portion 22 of the main body 2 does not cover at least the piston 32 and does not cover the entire switch 3, for example. The applicator 1C does not have a portion that covers the piston 32 from the side of the top portion 22 and does not have a portion that covers the entire switch 3 from the side of the top portion 22, for example. Therefore, when the applicator 1C is seen from the side of the top portion 22 in the height direction D1, the piston 32 is exposed to the outside of the applicator 1C.

The stopper 4 is a component that supports the microneedle device 11 which is disposed in the main body 2 to be located between the piston 32 and the skin. As described above, the applicator 1C includes one or more stoppers 4, for example, two stoppers 4. The two stoppers 4 may be provided to be line-symmetrical with respect to the imaginary center line (the center line CL1) of the arm 31 in the extension direction of the arm 31 as a reference axis. As an example, the two stoppers 4 are located to sandwich a vicinity of the free end 31b of the arm 31 in the width direction D3. The two stoppers 4 are provided along the outer edge of the microneedle device 11 in the set state. Each of the stoppers 4 is mounted over an inner surface of the bottom portion 21 that defines the opening 21a and an inner surface of the wall portion 26 that is continuous with the inner surface of the bottom portion 21. Each of the stoppers 4 has a columnar shape which extends in the height direction D1. As an example, a bottom surface of the stopper 4 is on the same plane as a bottom surface (a surface that comes into contact with the skin when the applicator 1C is placed on the skin) of the main body 2.

The stopper 4 overlaps the opening 21a and does not overlap the switch 3 when seen in the height direction D1. Therefore, when the switch 3 moves, the stopper 4 does not come into contact with the switch 3. The stopper 4 has a placement surface 4a on which the microneedle device 11 is disposed in the set state. The placement surface 4a is a surface facing upward and is inclined with respect to a plane orthogonal to the height direction D1. The placement surface 4a may be parallel to the plane orthogonal to the height direction D1.

The stopper 4 is provided closer to the arm 31 than the end portion 32b of the piston 32 on the center line CL1. The end portion 32b is also an end portion of the switch 3 on the center line CL1 and is located on the side opposite to the fixed end 31a. Therefore, the stopper 4 mainly supports a portion of the microneedle device 11 on the side of the arm 31 in the set state. As an example, the stopper 4 supports the peripheral edge portion 11c. The stopper 4 may support the platform surface 11d when it does not come into contact with the microneedles 11a.

When the arm 31 generates an elastic force, like an elastic member such as a leaf spring, the arm 31 deforms toward the top portion 22, and thus an elastic force is generated in the arm 31. As an example, the microneedle device 11 inserted into the main body 2 through the opening 21a pushes the piston 32 upward, thereby causing the arm 31 to deform toward the top portion 22. Alternatively, the arm 31 is deformed toward the top portion 22 by such an insertion operation by the user. The switch 3, for example, the piston 32, transmits the elastic force generated in the arm 31 to the microneedle device 11 supported by the stopper 4. At this time, the switch 3 comes into contact with the microneedle device 11. The arm 31 may come into contact with the microneedle device 11, or the piston surface 32a may come into contact with the microneedle device 11. The stopper 4 supports the microneedle device 11 against the elastic force. In this way, the microneedle device 11 is gripped by the switch 3 and the stopper 4 and thus supported against the elastic force.

The rib 5 is a component that supports the microneedle device 11 together with the stopper 4. The rib 5 is provided at a position different from that of the stopper 4. As an example, the rib 5 is located on the imaginary center line (the center line CL1) of the arm 31 in the extension direction of the arm 31. The rib 5 is provided closer to the end portion 32b than the arm 31. Therefore, in the set state, the rib 5 mainly supports a portion of the microneedle device 11 on the side of the end portion 32b. The rib 5 has an L shape that extends from the top portion 22 to the bottom portion 21 in the height direction D1 and then bends toward the center of the main body 2. A tip end surface 5a of the rib 5 faces the center of the main body 2. The rib 5 does not reach the bottom surface of the main body 2 in the height direction D1 and does not come into contact with the skin when the applicator 1C is placed on the skin. Since a gap is formed between the entire bottom surface and side surfaces of the rib 5 and other components of the main body 2, the rib 5 can bend in a direction orthogonal to the height direction D1. In the applicator 1C, the rib 5 extends downward from a portion of the first side portion 23 that protrudes toward the end portion 32b. As described above, the applicator 1C may have a single rib 5 or two or more ribs 5. For example, the two ribs 5 may be provided to be line-symmetrical with respect to the center line CL1 as the reference axis, as in the stopper 4.

The rib 5 comes into contact with at least the side surface 11e of the microneedle device 11 which is supported by the stopper 4. As an example, the tip end surface 5a of the rib 5 comes into contact with the side surface 11e and does not come into contact with any portion of the microneedle device 11 other than the side surface 11e. The rib 5 may push the microneedle device 11 toward the stopper 4. It can be said that the stopper 4 supports the microneedle device 11 from below, whereas the rib 5 supports the microneedle device 11 from the side.

The eaves portion 6 is a component that stops the movement of the microneedle device 11 within the main body 2 so that the microneedle device 11, which is inserted into the main body 2 through the opening 21a and moves toward the top portion 22, does not pop out above the main body 2. As described above, the applicator 1C has one or more eaves portions 6, for example, two eaves portions 6. As an example, the two eaves portions 6 are provided to be line-symmetrical with respect to the imaginary center line (the center line CL1) of the arm 31 in the extension direction of the arm 31 as a reference axis. The two eaves portions 6 are located to sandwich the piston 32 in the width direction D3 when seen in the height direction D1. The eaves portions 6 overlap the opening 21a and do not overlap the switch 3 when seen in the height direction D1. Thus, when the switch 3 moves, the eaves portion 6 does not come into contact with the switch 3. In the applicator 1C, when seen in the height direction D1, most of the eaves portions 6 extend in the lateral direction D2 and both end portions of the eaves portions 6 are bent. The eaves portions 6 may be formed integrally with the wall portion 26.

The eaves portions 6 are provided closer to the top portion 22 than the placement surface 4a of the stopper 4 in the height direction D1. In other words, the eaves portions 6 are located above the stopper 4. A distance between lower ends 6a of the eaves portions 6 and the upper end 4b of the placement surface 4a in the height direction D1 is greater than the thickness of the peripheral edge portion 11c of the microneedle device 11. From the viewpoint of holding the microneedle device 11 more stably, the distance between the lower ends 6a of the eaves portions 6 and the upper end 4b of the placement surface 4a may be the same as or smaller than the thickness of the peripheral edge portion 11c of the microneedle device 11. The lower ends 6a are ends of the eaves portions 6 that are located on the side of the bottom portion 21 in the height direction D1. The upper end 4b is an end of the placement surface 4a that is located on the side of the top portion 22 in the height direction D1. In the applicator 1C, the upper end 4b is an upper end of the stopper 4.

The eaves portions 6 are provided to cover at least a part of the peripheral edge portion 11c of the microneedle device 11 when the microneedle device 11 supported by the stopper 4 is seen from the side of the top portion 22 in the height direction D1. As an example, the eaves portions 6 cover a part of the peripheral edge portion 11c. As an example, the eaves portions 6 are provided partially along the peripheral edge portion 11c so that other portions of the peripheral edge portion 11c are visible when the microneedle device 11 is seen from the side of the top portion 22 in the height direction D1. In the applicator 1C, the eaves portions 6 are provided at two locations along the peripheral edge portion 11c.

The recovery mechanism 7 is a component that holds the microneedle device 11, at least when the microneedle device 11 is applied to the skin by an operation of the switch 3 by the user. The recovery mechanism 7 has two gripping claws 71 that grip the microneedle device 11. As an example, the gripping claws 71 grip the microneedle device 11 at approximately the same time as the microneedle device 11 reaches the skin in response to the piston 32 moving toward the skin.

In the applicator 1C, the recovery mechanism 7 is a plurality of L-shaped members located outside the piston 32. As an example, the recovery mechanism 7 is two L-shaped members located on both sides of the piston 32 in the width direction D3. Each of the L-shaped members has a horizontal portion 72 that extends in the width direction D3 and a vertical portion 73 that extends in the height direction D1 to the top portion 22. The horizontal portion 72 is a bar-shaped portion that extends from the inner surface of the first side portion 23 toward the inside of the main body 2. As an example, the horizontal portion 72 comes into contact with the skin when the applicator 1C is placed on the skin. The horizontal portion 72 has an inner end 72a located inside the main body 2. The inner end 72a is located near the piston 32. The vertical portion 73 is a bar-shaped portion that extends from the inner end 72a toward the top portion 22. The vertical portion 73 has an upper end 73a located on the side of the top portion 22. The upper end 73a is located outside the eaves portions 6 in the width direction D3. In other words, the eaves portions 6 are provided inside the main body 2 with respect to the upper end 73a. In the applicator 1C, each of the gripping claws 71 is provided at the inner end 72a to protrude from the inner end 72a toward the piston 32.

As an example, an angle formed by the horizontal portion 72 and the vertical portion 73 is 90 degrees. The angle does not have to be 90 degrees as long as the vertical portion 73 can securely apply the microneedle device 11 to the skin without interfering with the microneedle device 11 and can hold the microneedle device 11. As an example, the angle may be 60 degrees or more and 120 degrees or less, or 80 degrees or more and 100 degrees or less.

Next, referring to FIG. 17, an operation until the microneedle device 11 is applied to the skin will be described.

In the set state, the microneedle device 11 is supported from below by the stopper 4 and from the side by the rib 5. At this time, the piston 32 of the switch 3 is located above the microneedle device 11. As the user pushes the switch 3 toward the skin, the microneedle device 11 pushed by the piston 32 moves downward while sliding on the placement surface 4a. At this time, since the rib 5 bends outward in the direction orthogonal to the height direction D1, the microneedle device 11 moves smoothly downward. As the user pushes the switch 3 further in, the microneedle device 11 pushed by the piston 32 moves further downward with respect to the placement surface 4a and reaches the skin. Thus, the microneedles 11a come into contact with or pierce the skin. In this way, the microneedle device 11 is applied to the skin.

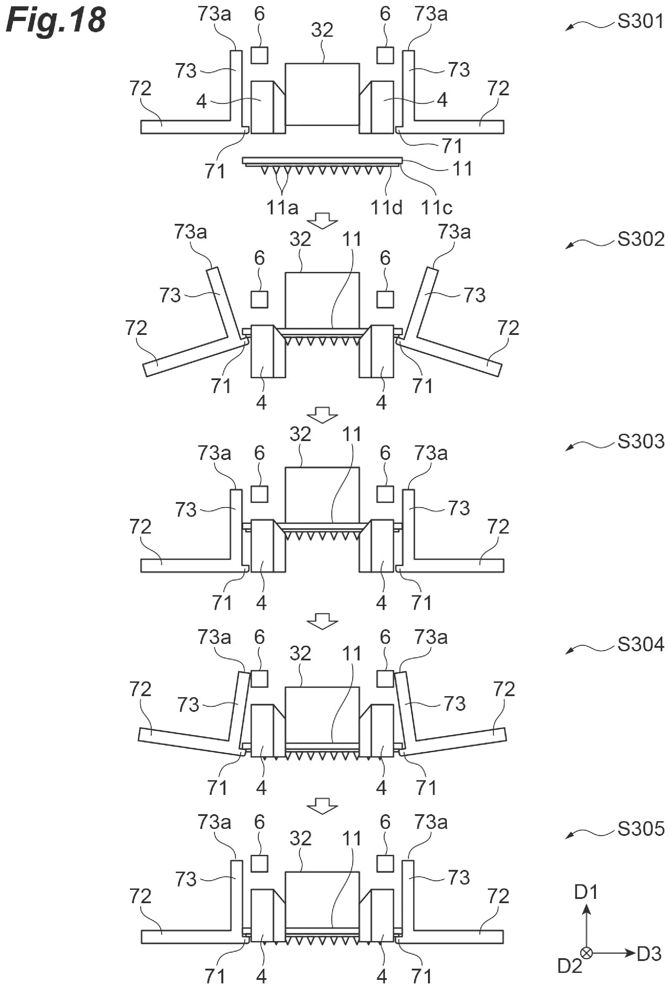

Next, with reference to FIG. 18, an operation until the gripping claws 71 grip the microneedle device 11 will be described.

In a pre-set state shown as scene S301, the microneedle device 11 is disposed below the applicator 1C.

In scene S302, the user inserts the microneedle device 11 into the main body 2. At this time, the microneedle device 11 comes into contact with the gripping claws 71 from below, and thus the vertical portion 73 tilts toward the outside of the main body 2. Once the microneedle device 11 has passed over the gripping claws 71, the vertical portion 73 returns to its original position. By the insertion operation described above, the microneedle device 11 enters between the piston 32 and the stopper 4 and is supported by the stopper 4, as shown in scene S303. The state shown in scene S303 is the set state shown in FIG. 17.

In scene S304, the user applies the microneedle device 11 to the skin, as described with reference to FIG. 17. When the piston 32 is pushed in toward the skin by the user, the microneedle device 11 reaches the skin. At about the same time, the gripping claws 71 grip the microneedle device 11 in the main body 2. When the piston 32 continues to be pushed in by the user in this state, the vertical portion 73 begins to tilt toward the inside of the main body 2. In the applicator 1C, however, since the eaves portions 6 are provided inside the main body 2 with respect to the upper end 73a, an extent to which the vertical portion 73 tilts toward the inside of the main body 2 is limited. Thus, since the vertical portion 73 is no longer excessively tilted toward the inside of the main body 2, the microneedle device 11 remains inside the main body 2. The gripping claws 71 may grip the peripheral edge portion 11c. Alternatively, the gripping claws 71 may grip the platform surface 11d when they do not come into contact with the microneedles 11a.

In scene S305, the user finishes the pushing-in of the piston 32, and the vertical portion 73 returns to its original position. The gripping claws 71 grip the microneedle device 11 even after the microneedle device 11 has been applied to the skin. Therefore, when the user moves the applicator 1C away from the skin, the microneedle device 11 gripped by gripping claws 71 is also moved away from the skin together with the applicator 1C.

As described above, the gripping claws 71 grip the microneedle device 11 at about the same time that the microneedle device 11 reaches the skin. As another example, the gripping claws 71 may grip the microneedle device 11 before or after the microneedle device 11 reaches the skin.

As described above, in the applicator 1C, the eaves portions 6 function to stop the movement of the microneedle device 11 toward the top portion 22, as well as to restrict the vertical portion 73 tilting toward the inside of the main body 2.

### [Application Kit According to Third Example]

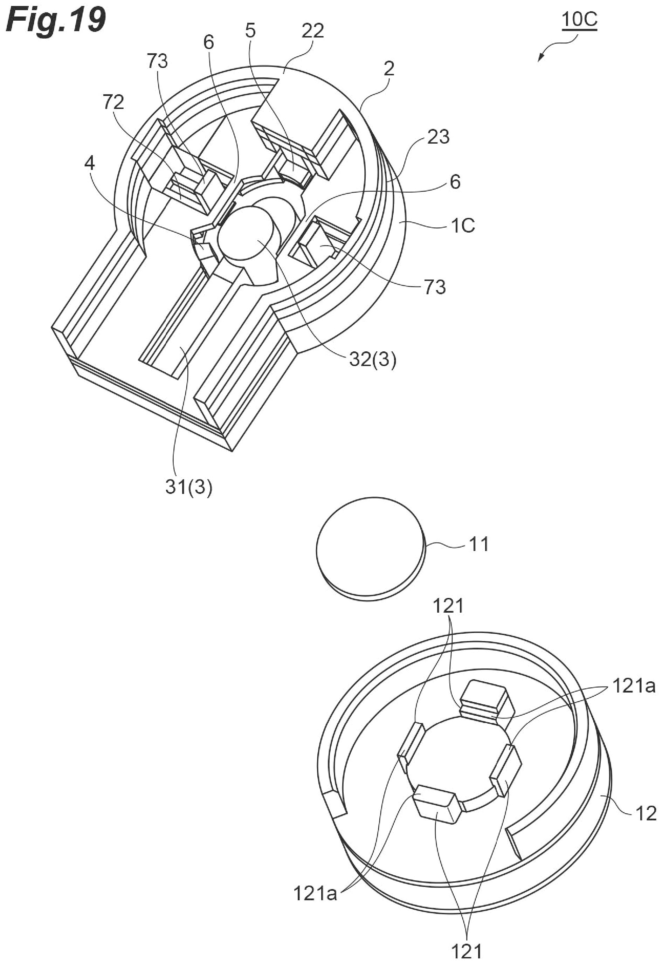

With reference to FIG. 19, a configuration of an application kit 10C according to the third example will be described. FIG. 19 shows a perspective view of the application kit 10C seen from above.

The stage 12 is an article for arranging the microneedle device 11 in the applicator 1C. The stage 12 has a columnar portion 121 that extends in the height direction D1 to support the microneedle device 11. The columnar portion 121 is provided partially along the peripheral edge portion 11c of the microneedle device 11. The columnar portion 121 has a top surface 121a that supports the microneedle device 11. The top surface 121a is a surface that faces the microneedle device 11. As an example, the top surface 121a supports the peripheral edge portion 11c and does not come into contact with the platform surface 11d.

In the application kit 10C, a process until the microneedle device 11 disposed on the stage 12 is disposed into the applicator 1C is the same as the process in the application kit 10A described with reference to FIG. 7.

### [Applicator According to Fourth Example]

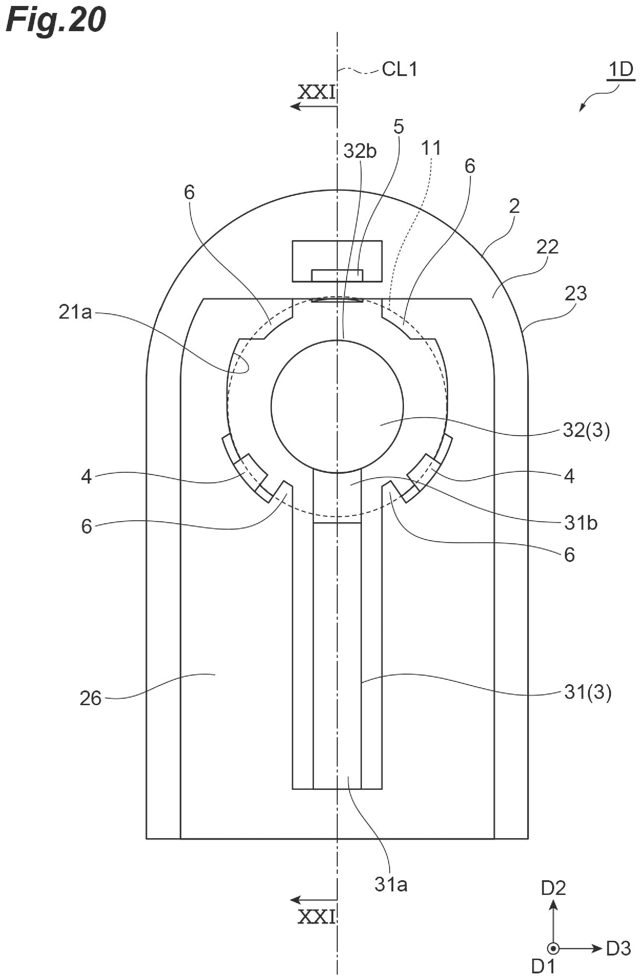

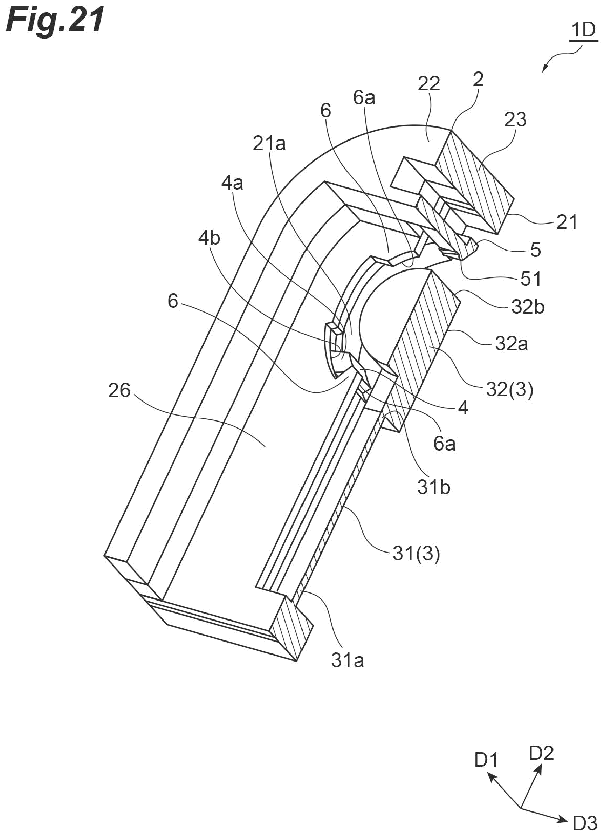

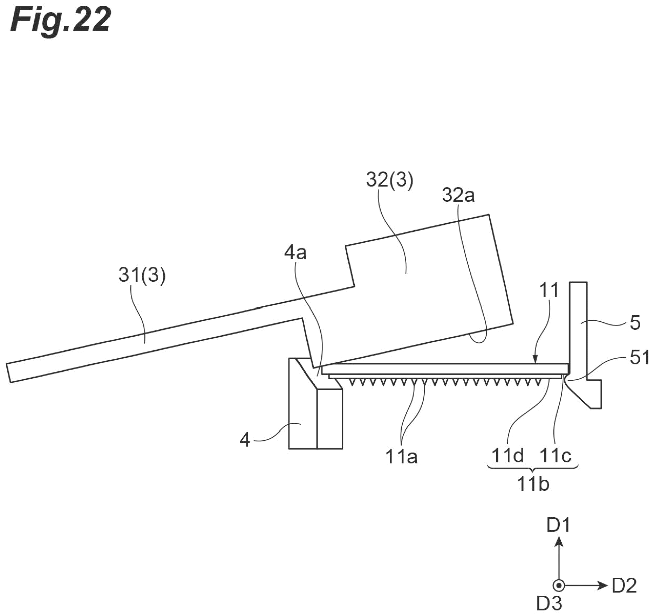

Referring to FIGS. 20 to 22, a structure of an applicator 1D according to a fourth example will be described. FIG. 20 is a plan view of the applicator 1D seen from above. FIG. 21 is a perspective cross-sectional view along line XXI-XXI shown in FIG. 20. FIG. 22 is a diagram showing a state in which the microneedle device is set in the applicator 1D. FIGS. 20 and 21 show the applicator 1D in the natural state.

The applicator 1D includes a main body 2, a switch 3, at least one stopper 4, at least one rib 5, and at least one eaves portion 6. A material of the applicator 1D is the same as that of the applicator 1A, for example. A configuration of the microneedle device 11 set in the applicator 1D is the same as that of the microneedle device 11 set in the applicator 1A.

The main body 2 is a structure that accommodates each component, such as the switch 3, and has a substantially rectangular parallelepiped shape with two rounded corners. The main body 2 has a bottom portion 21, a top portion 22, a first side portion 23 (a side portion) and a wall portion 26. The bottom portion 21 is a portion that comes into contact with the skin when the applicator 1D is placed on the skin to apply the microneedle device 11 to the skin. The bottom portion 21 has a substantially rectangular shape with two rounded corners. An opening 21a for inserting the microneedle device 11 into the main body 2 is formed in a substantially center of the bottom portion 21. The top portion 22 is a portion opposite to the bottom portion 21 and is thus located away from that skin when the applicator 1D is placed on the skin.

Definitions of height direction D1, lateral direction D2, and width direction D3 used in the following description are the same as the definitions of the height direction D1, the lateral direction D2, and the width direction D3 used in the description of the first example. Further, the side of the top portion 22 in the height direction D1 may be referred to as the upper side of the applicator 1D, and the side of the bottom portion 21 in the height direction D1 may be referred to as the lower side of the applicator 1D.

The first side portion 23 extends in the height direction D1 and defines an outer circumference of the main body 2. As an example, the first side portion 23 extends in the circumferential direction of the bottom portion 21 and in the height direction D1 from the entire outer edge of the bottom portion 21. A top surface of the first side portion 23 may be present or function as the top portion 22. As an example, in order to improve user convenience, a height of a portion of the first side portion 23 from the bottom portion 21 (for example, a portion on the side of a fixed end 31a of an arm 31) may be lower than a height of the other portion of the first side portion 23 from the bottom portion 21.

The wall portion 26 extends from the entire inner surface of the first side portion 23 toward the inside of the main body 2. In the applicator 1D, the wall portion 26 is integrally formed with the bottom portion 21. As an example, the wall portion 26 is a thicker portion in the height direction D1 than the bottom portion 21.

As an example, the main body 2 is formed to be line-symmetrical with respect to the imaginary center line CL1 parallel to the lateral direction D2 as the reference axis. In other words, each of the bottom portion 21, the top portion 22, the first side portion 23, and the wall portion 26 is formed to be line-symmetrical with respect to the center line CL1 as a reference axis.

The switch 3 is a component that is operated by the user to push the microneedle device 11 toward the skin. The switch 3 has an arm 31 and a piston 32. The arm 31 is a rectangular plate-shaped portion mounted on the first side portion 23 to extend in the lateral direction D2. Therefore, the lateral direction D2 may also be referred to as an extension direction of the arm 31. The arm 31 has a fixed end 31a mounted on the first side portion 23, and a free end 31b located on the side opposite to the fixed end 31a. The arm 31 can move in an arc shape in the height direction D1 with the fixed end 31a as a fulcrum, and this movement allows the free end 31b to move in the height direction D1. The arm 31 may be formed of a member or material that generates an elastic force, and may be, for example, an elastic member such as a leaf spring. Alternatively, the arm 31 may be a non-elastic member or a flexible member. The piston 32 is a plate-shaped portion mounted on the free end 31b. The piston 32 has a piston surface 32a which is a surface facing the skin. In one example, the switch 3 is formed to be line-symmetrical with respect to the center line CL1 as a reference axis. As an example, the arm 31 extends parallel to the lateral direction D2 when seen in the width direction D3. The arm 31 may extend to be inclined with respect to the lateral direction D2 when seen in the width direction D3.

In the set state, the microneedle device 11 is located between the piston 32 and the bottom portion 21. The microneedle device 11 is disposed in the main body 2 so that the microneedle device 11 is located between the piston 32 and the skin when the bottom portion 21 comes into contact with the skin. In other words, it can be said that the piston 32, the microneedle device 11, and the bottom portion 21 (the skin) are arranged in this order in the height direction D1. The piston 32 is pushed in by the user toward the skin when the microneedle device 11 is applied to the skin. In other words, the piston 32 is pushed in by the user to the side of the bottom portion 21 in the height direction D1. At this time, the arm 31 moves in an arc shape with the fixed end 31a as a fulcrum in conjunction with the movement of the piston 32. The piston surface 32a comes into contact with the microneedle device 11 when it is pushed in by the user toward the skin. The piston surface 32a may or may not be in contact with the microneedle device 11 in the set state.

When the applicator 1D is seen in the height direction D1, the entire switch 3 is located inside opening 21a. Thus, the switch 3 does not come into contact with bottom portion 21 when the piston 32 is pushed in by the user toward the skin.

When the applicator 1D is seen from the side of top portion 22 in the height direction D1, the top portion 22 of the main body 2 does not cover at least the piston 32 and does not cover the entire switch 3, for example. The applicator 1D does not have a portion that covers the piston 32 from the side of the top portion 22 and does not have a portion that covers the entire switch 3 from the side of the top portion 22, for example. Therefore, when the applicator 1D is seen from the side of the top portion 22 in the height direction D1, the piston 32 is exposed to the outside of the applicator 1D.

The stopper 4 is a component that supports the microneedle device 11 which is disposed in the main body 2 to be located between the piston 32 and the skin. As described above, the applicator 1D includes one or more stoppers 4, for example, two stoppers 4. The two stoppers 4 may be provided to be line-symmetrical with respect to the imaginary center line (the center line CL1) of the arm 31 in the extension direction of the arm 31 as a reference axis. As an example, the two stoppers 4 are located to sandwich a vicinity of the free end 31b of the arm 31 in the width direction D3. The two stoppers 4 are provided along the outer edge of the microneedle device 11 in the set state. Each of the stoppers 4 is mounted over an inner surface of the bottom portion 21 that defines the opening 21a and an inner surface of the wall portion 26 that is continuous with the inner surface of the bottom portion 21. Each of the stoppers 4 has a columnar shape which extends in the height direction D1. As an example, a bottom surface of the stopper 4 is located on the same plane as a bottom surface (a surface that comes into contact with the skin when the applicator 1D is placed on the skin) of the main body 2.

The stopper 4 overlaps the opening 21a and does not overlap the switch 3 when seen in the height direction D1. Therefore, when the switch 3 moves, the stopper 4 does not come into contact with the switch 3. The stopper 4 has a placement surface 4a on which the microneedle device 11 is disposed in the set state. The placement surface 4a is a surface facing upward and is inclined with respect to a plane orthogonal to the height direction D1. The placement surface 4a may be parallel to the plane orthogonal to the height direction D1.

The stopper 4 is provided closer to the arm 31 than the end portion 32b of the piston 32 on the center line CL1. The end portion 32b is also an end portion of the switch 3 on the center line CL1 and is located on the side opposite to the fixed end 31a. Therefore, the stopper 4 mainly supports a portion of the microneedle device 11 on the side of the arm 31 in the set state. As an example, the stopper 4 supports the peripheral edge portion 11c. The stopper 4 may support the platform surface 11d when it does not come into contact with the microneedles 11a.

When the arm 31 generates an elastic force, like an elastic member such as a leaf spring, the arm 31 deforms toward the top portion 22, and thus an elastic force is generated in the arm 31. As an example, the microneedle device 11 inserted into the main body 2 through the opening 21a pushes the piston 32 upward, thereby causing the arm 31 to deform toward the top portion 22. Alternatively, the arm 31 is deformed toward the top portion 22 by such an insertion operation by the user. The switch 3, for example, the piston 32, transmits the elastic force generated in the arm 31 to the microneedle device 11 supported by the stopper 4. At this time, the switch 3 comes into contact with the microneedle device 11. The arm 31 may come into contact with the microneedle device 11, or the piston surface 32a may come into contact with the microneedle device 11. The stopper 4 supports the microneedle device 11 against the elastic force. In this way, the microneedle device 11 is gripped by the switch 3 and the stopper 4 and thus supported against the elastic force.

The rib 5 is a component that supports the microneedle device 11 together with the stopper 4. The rib 5 is provided at a position different from that of the stopper 4. As an example, the rib 5 is located on the imaginary center line (the center line CL1) of the arm 31 in the extension direction of the arm 31. The rib 5 is provided closer to the end portion 32b than the arm 31. Therefore, in the set state, the rib 5 mainly supports a portion of the microneedle device 11 on the side of the end portion 32b. The rib 5 extends from the top portion 22 to the bottom portion 21 in the height direction D1. The rib 5 does not reach the bottom surface of the main body 2 in the height direction D1 and does not come into contact with the skin when the applicator 1D is placed on the skin. A protrusion 51 that protrudes toward the end portion 32b of the piston 32 when seen in the height direction D1 is provided on the rib 5. Since a gap is formed between the entire bottom surface and side surfaces of the rib 5 and other components of the main body 2, the rib 5 can bend in a direction orthogonal to the height direction D1. As described above, the applicator 1D may have a single rib 5 or two more ribs 5. For example, the two ribs 5 may be provided to be line-symmetrical with respect to the center line CL1 as the reference axis, as in the stopper 4.

The rib 5 comes into contact with at least the side surface 11e of the microneedle device 11 which is supported by the stopper 4. The protrusion 51 of the rib 5 supports the peripheral edge portion 11c from below. Thus, the microneedle device 11 can be held more stably. The rib 5 may push the microneedle device 11 to the side of the stopper 4. It can be said that the stopper 4 supports the microneedle device 11 from below, whereas the rib 5 supports the microneedle device 11 from the side and below. The protrusion 51 of the rib 5 may support the platform surface 11d from below when it does not come into contact with the microneedles 11a.

The eaves portion 6 is a component that stops the movement of the microneedle device 11 within the main body 2 so that the microneedle device 11, which is inserted into the main body 2 through the opening 21a and moves toward the top portion 22, does not pop out above the main body 2. As described above, the applicator 1D has one or more eaves portions 6, for example, four eaves portions 6. As an example, the four eaves portions 6 are provided to be line-symmetrical with respect to an imaginary center line (the center line CL1) of the arm 31 in the extension direction of the arm 31 as a reference axis. The two eaves portions 6 are provided closer to the end portion 32b of the piston 32 than to the arm 31. The other two eaves portions 6 are provided closer to the arm 31 than the end portion 32b. When seen in the height direction D1, the other two eaves portions 6 are located inside the main body 2 with respect to the stopper 4 and are located to sandwich a vicinity of the free end 31b of the arm 31. The eaves portions 6 overlap the opening 21a and do not overlap the switch 3 when seen in the height direction D1. Thus, when the switch 3 moves, the eaves portions 6 do not come into contact with the switch 3. The eaves portions 6 may be formed integrally with the wall portion 26.

The eaves portions 6 are provided closer to the top portion 22 than the placement surface 4a of the stopper 4 in the height direction D1. In other words, the eaves portions 6 are located above the stopper 4. A distance between lower ends 6a of the eaves portions 6 and the upper end 4b of the placement surface 4a in the height direction D1 is greater than the thickness of the peripheral edge portion 11c of the microneedle device 11. From the viewpoint of holding the microneedle device 11 more stably, the distance between the lower ends 6a of the eaves portions 6 and the upper end 4b of the placement surface 4a may be the same as or smaller than the thickness of the peripheral edge portion 11c of the microneedle device 11. The lower ends 6a are ends of the eaves portions 6 that are located on the side of the bottom portion 21 in the height direction D1. The upper end 4b is an end of the placement surface 4a that is located on the side of the top portion 22 in the height direction D1. In the applicator 1D, the upper end 4b is an upper end of the stopper 4.

The eaves portions 6 are provided to cover at least a part of the peripheral edge portion 11c of the microneedle device 11 when the microneedle device 11 supported by the stopper 4 is seen from the side of the top portion 22 in the height direction D1. As an example, the eaves portions 6 cover a part of the peripheral edge portion 11c. As an example, the eaves portions 6 are provided partially along the peripheral edge portion 11c so that other portions of the peripheral edge portion 11c are visible when the microneedle device 11 is seen from the side of the top portion 22 in the height direction D1. In the applicator 1D, the eaves portions 6 are provided at four locations along the peripheral edge portion 11c.

Next, referring to FIG. 22, a series of operations until the microneedle device 11 is applied to the skin will be described.

In the set state, the microneedle device 11 is supported from below by the stopper 4 and from the side and below by the rib 5. At this time, the piston 32 of the switch 3 is located above the microneedle device 11. As the user pushes the switch 3 toward the skin, the stopper 4 and the protrusion 51 of the rib 5 continue to support the microneedle device 11. When the microneedle device 11 pushed by the piston 32 passes over the protrusion 51, the microneedle device 11 moves downward while sliding on the placement surface 4a. At this time, since the rib 5 bends outward in the direction orthogonal to the height direction D1, the microneedle device 11 moves smoothly downward.

As the user pushes the switch 3 further in, the microneedle device 11 pushed by the piston 32 moves further down with respect to the placement surface 4a and reaches the skin. Thus, the microneedles 11a come into contact with or pierce the skin. In this way, the microneedle device 11 is applied to the skin. As described above, in the applicator 1D, the user needs to push the switch 3 firmly in order for the microneedle device 11 to pass over the protrusion 51. Thus, the microneedle device 11 can reach the skin more reliably.

### [Application Kit According to Fourth Example]

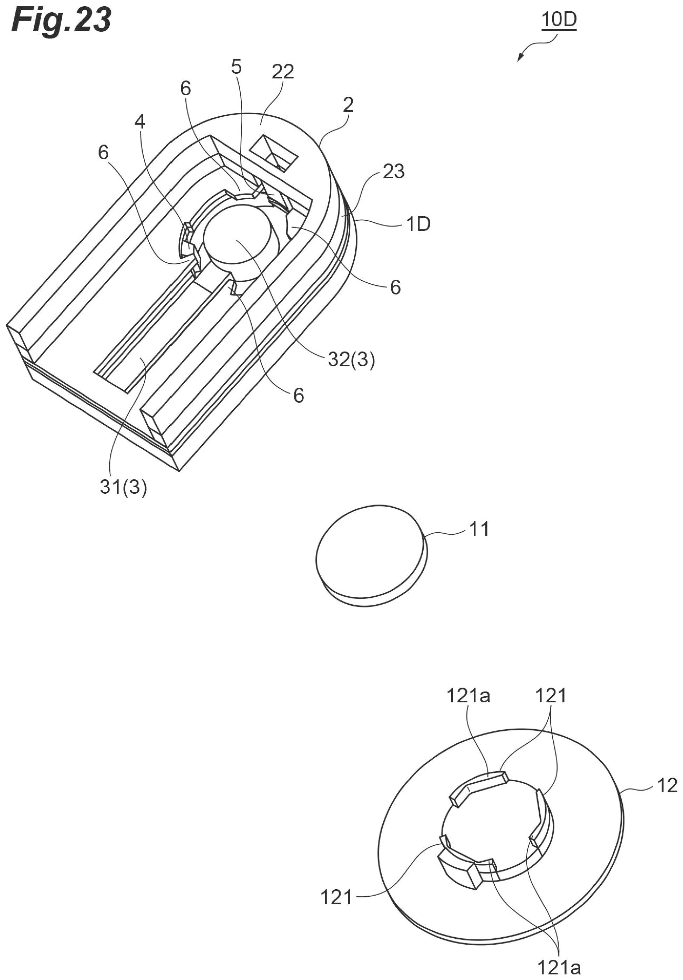

With reference to FIG. 23, a configuration of an application kit 10D according to the fourth example will be described. FIG. 23 is a perspective view of the application kit 10D seen from above.

The stage 12 is an article for arranging the microneedle device 11 in the applicator 1D. The stage 12 has a columnar portion 121 that extends in the height direction D1 to support the microneedle device 11. The columnar portion 121 is provided partially along the peripheral edge portion 11c of the microneedle device 11. The columnar portion 121 has a top surface 121a that supports the microneedle device 11. The top surface 121a is a surface that faces the microneedle device 11. As an example, the top surface 121a supports the peripheral edge portion 11c and does not come into contact with the platform surface 11d.

In the application kit 10D, a process until the microneedle device 11 disposed on the stage 12 is disposed into the applicator 1D is the same as the process in the application kit 10A described with reference to FIG. 7.

### [Applicator According to Fifth Example]

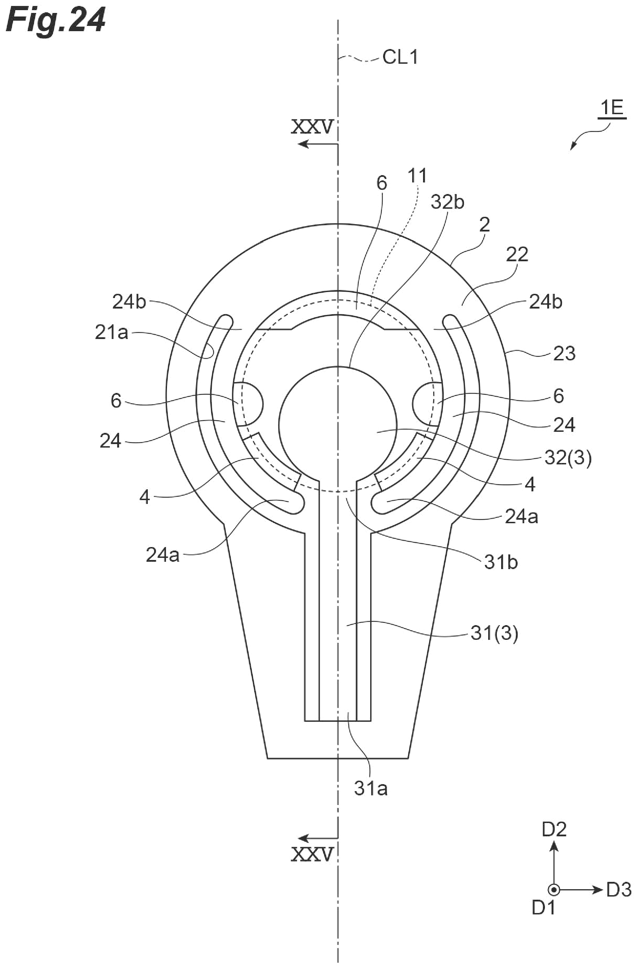

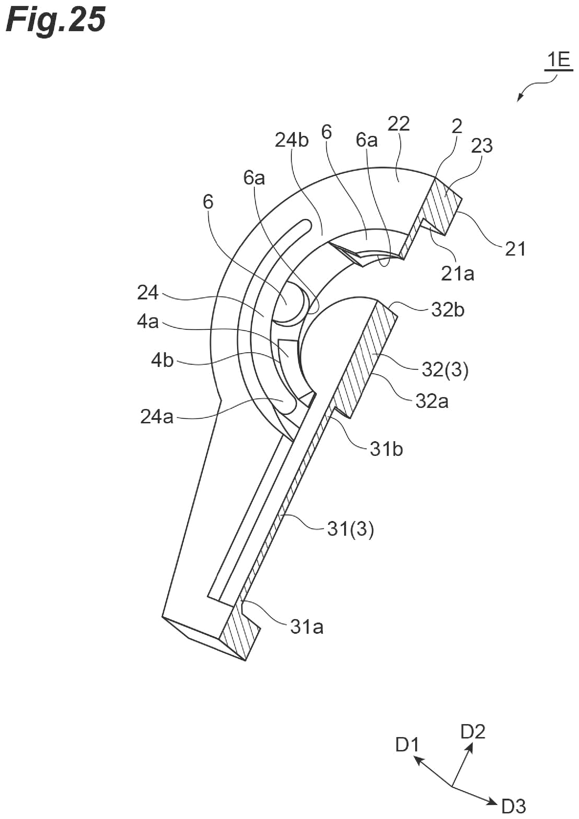

Referring to FIGS. 24 and 25, a structure of an applicator 1E according to a fifth example will be described. FIG. 24 is a plan view of the applicator 1E seen from above. FIG. 25 is a perspective cross-sectional view along line XXV-XXV shown in FIG. 24. FIGS. 24 and 25 show the applicator 1E in the natural state.

The applicator 1E includes a main body 2, a switch 3, at least one stopper 4, and at least one eaves portion 6. A material of the applicator 1E is the same as that of the applicator 1A, for example. A configuration of the microneedle device 11 set in the applicator 1E is the same as that of the microneedle device 11 set in the applicator 1A.

The main body 2 is a structure that accommodates each component, such as the switch 3, and is formed by a combination of a substantially cylindrical portion and a substantially quadrangular frustum-shaped portion. The main body 2 has a bottom portion 21, a top portion 22, a first side portion 23 (a side portion) and a second side portion 24. The bottom portion 21 is a portion that comes into contact with the skin when the applicator 1E is placed on the skin to apply the microneedle device 11 to the skin. The bottom portion 21 is formed by a combination of a substantially circular portion and a substantially trapezoidal portion. An opening 21a for inserting the microneedle device 11 into the main body 2 is formed in a substantially center of the bottom portion 21. The top portion 22 is a portion opposite to the bottom portion 21 and is thus located away from that skin when the applicator 1E is placed on the skin.

Definitions of height direction D1, lateral direction D2, and width direction D3 used in the following description are the same as the definitions of the height direction D1, the lateral direction D2, and the width direction D3 used in the description of the first example. Further, the side of the top portion 22 in the height direction D1 may be referred to as the upper side of the applicator 1E, and the side of the bottom portion 21 in the height direction D1 may be referred to as the lower side of the applicator 1E.

The first side portion 23 extends in the height direction D1 and defines an outer circumference of the main body 2. As an example, the first side portion 23 extends in the circumferential direction of the bottom portion 21 and in the height direction D1 from the entire outer edge of the bottom portion 21. A top surface of the first side portion 23 may be present or function as the top portion 22. As an example, in order to improve user convenience, a height of a portion of the first side portion 23 from the bottom portion 21 (for example, a portion on the side of a fixed end 31a of an arm 31) may be lower than a height of the other portion of the first side portion 23 from the bottom portion 21.

The second side portion 24 is connected to the first side portion 23 inside the first side portion 23. The second side portion 24 extends in an arc shape so that a gap is formed between the second side portion 24 and the first side portion 23. The second side portion 24 extends along the peripheral edge portion 11c of the microneedle device 11 in the set state. Since a gap is formed between the entire surface of the second side portion 24 and the first side portion 23, the second side portion 24 can bend toward the first side portion 23.

As an example, the main body 2 is formed to be line-symmetrical with respect to the imaginary center line CL1 parallel to the lateral direction D2 as the reference axis. In other words, each of the bottom portion 21, the top portion 22, the first side portion 23, and the second side portion 24 is formed to be line-symmetrical with respect to the center line CL1 as a reference axis.

The switch 3 is a component that is operated by the user to push the microneedle device 11 toward the skin. The switch 3 has an arm 31 and a piston 32. The arm 31 is a rectangular plate-shaped portion mounted on the first side portion 23 to extend in the lateral direction D2. Therefore, the lateral direction D2 may also be referred to as an extension direction of the arm 31. The arm 31 has a fixed end 31a mounted on the first side portion 23, and a free end 31b located on the side opposite to the fixed end 31a. Free ends 24a of the second side portion 24 are located on both sides of the free end 31b in the width direction D3. The arm 31 can move in an arc shape in the height direction D1 with the fixed end 31a as a fulcrum, and this movement allows the free end 31b to move in the height direction D1. The arm 31 may be formed of a member or material that generates an elastic force, and may be, for example, an elastic member such as a leaf spring. Alternatively, the arm 31 may be a non-elastic member or a flexible member. The piston 32 is a plate-shaped portion mounted on the free end 31b. The piston 32 has a piston surface 32a which is a surface facing the skin. In one example, the switch 3 is formed to be line-symmetrical with respect to the center line CL1 as a reference axis. As an example, the arm 31 extends parallel to the lateral direction D2 when seen in the width direction D3. The arm 31 may extend to be inclined with respect to the lateral direction D2 when seen in the width direction D3.

In the set state, the microneedle device 11 is located between the piston 32 and the bottom portion 21. The microneedle device 11 is disposed in the main body 2 so that the microneedle device 11 is located between the piston 32 and the skin when the bottom portion 21 comes into contact with the skin. In other words, it can be said that the piston 32, the microneedle device 11, and the bottom portion 21 (the skin) are arranged in this order in the height direction D1. The piston 32 is pushed in by the user toward the skin when the microneedle device 11 is applied to the skin. In other words, the piston 32 is pushed in by the user to the side of the bottom portion 21 in the height direction D1. At this time, the arm 31 moves in an arc shape with the fixed end 31a as a fulcrum in conjunction with the movement of the piston 32. The piston surface 32a comes into contact with the microneedle device 11 when it is pushed in by the user toward the skin. The piston surface 32a may or may not be in contact with the microneedle device 11 in the set state.

When the applicator 1E is seen in the height direction D1, the entire switch 3 is located inside opening 21a. Thus, the switch 3 does not come into contact with bottom portion 21 when the piston 32 is pushed in by the user toward the skin.

When the applicator 1E is seen from the side of top portion 22 in the height direction D1, the top portion 22 of the main body 2 does not cover at least the piston 32 and does not cover the entire switch 3, for example. The applicator 1E does not have a portion that covers the piston 32 from the side of the top portion 22 and does not have a portion that covers the entire switch 3 from the side of the top portion 22, for example. Therefore, when the applicator 1E is seen from the side of the top portion 22 in the height direction D1, the piston 32 is exposed to the outside of the applicator 1E.

The stopper 4 is a component that supports the microneedle device 11 which is disposed in the main body 2 to be located between the piston 32 and the skin. As described above, the applicator 1E includes one or more stoppers 4, for example, two stoppers 4. The two stoppers 4 may be provided to be line-symmetrical with respect to the imaginary center line (the center line CL1) of the arm 31 in the extension direction of the arm 31 as a reference axis. As an example, the two stoppers 4 are located to sandwich a vicinity of the free end 31b of the arm 31 in the width direction D3. In the applicator 1E, the two stoppers 4 are provided closer to the free end 24a than the fixed end 24b of the second side portion 24. The two stoppers 4 are provided along the outer edge of the microneedle device 11 in the set state. Each of the stoppers 4 protrudes from the inner surface of the second side portion 24 toward the inside of the main body 2.

The stopper 4 overlaps the opening 21a and does not overlap the switch 3 when seen in the height direction D1. Therefore, when the switch 3 moves, the stopper 4 does not come into contact with the switch 3. The stopper 4 has a placement surface 4a on which the microneedle device 11 is disposed in the set state. The placement surface 4a is a surface facing upward and is inclined with respect to a plane orthogonal to the height direction D1. The placement surface 4a may be parallel to the plane orthogonal to the height direction D1.

The stopper 4 is provided closer to the arm 31 than the end portion 32b of the piston 32 on the center line CL1. The end portion 32b is also an end portion of the switch 3 on the center line CL1 and is located on the side opposite to the fixed end 31a. Therefore, the stopper 4 mainly supports a portion of the microneedle device 11 on the side of the arm 31 in the set state. As an example, the stopper 4 supports the peripheral edge portion 11c. The stopper 4 may support the platform surface 11d when it does not come into contact with the microneedles 11a.

When the arm 31 generates an elastic force, like an elastic member such as a leaf spring, the arm 31 deforms toward the top portion 22, and thus an elastic force is generated in the arm 31. As an example, the microneedle device 11 inserted into the main body 2 through the opening 21a pushes the piston 32 upward, thereby causing the arm 31 to deform toward the top portion 22. Alternatively, the arm 31 is deformed toward the top portion 22 by such an insertion operation by the user. The switch 3, for example, the piston 32, transmits the elastic force generated in the arm 31 to the microneedle device 11 supported by the stopper 4. At this time, the switch 3 comes into contact with the microneedle device 11. The arm 31 may come into contact with the microneedle device 11, or the piston surface 32a may come into contact with the microneedle device 11. The stopper 4 supports the microneedle device 11 against the elastic force. In this way, the microneedle device 11 is gripped by the switch 3 and the stopper 4 and thus supported against the elastic force.

The eaves portion 6 is a component that stops the movement of the microneedle device 11 within the main body 2 so that the microneedle device 11, which is inserted into the main body 2 through the opening 21a and moves toward the top portion 22, does not pop out above the main body 2. As described above, the applicator 1E has one or more eaves portions 6, for example, three eaves portions 6. As an example, one eaves portion 6 is located on an imaginary center line (the center line CL1) of the arm 31 in the extension direction of the arm 31 and is provided on the first side portion 23. The other two eaves portions 6 are located to be line-symmetrical with respect to the imaginary center line (the center line CL1) of the arm 31 as the reference axis and are provided on the second side portion 24. The two eaves portions 6 are located at the center of the second side portion 24 to be away from the free end 24a of the second side portion 24. The three eaves portions 6 protrude from the first side portion 23 or the second side portion 24 toward the inside of the main body 2 when seen in the height direction D1. The eaves portions 6 overlap the opening 21a and do not overlap the switch 3 when seen in the height direction D1. Thus, when the switch 3 moves, the eaves portions 6 do not come into contact with the switch 3.

The eaves portions 6 are provided closer to the top portion 22 than the placement surface 4a of the stopper 4 in the height direction D1. In other words, the eaves portions 6 are located above the stopper 4. A distance between lower ends 6a of the eaves portions 6 and the upper end 4b of the placement surface 4a in the height direction D1 is greater than the thickness of the peripheral edge portion 11c of the microneedle device 11. From the viewpoint of holding the microneedle device 11 more stably, the distance between the lower ends 6a of the eaves portions 6 and the upper end 4b of the placement surface 4a may be the same as or smaller than the thickness of the peripheral edge portion 11c of the microneedle device 11. The lower ends 6a are ends of the eaves portions 6 that are located on the side of the bottom portion 21 in the height direction D1. The upper end 4b is an end of the placement surface 4a that is located on the side of the top portion 22 in the height direction D1. In the applicator 1E, the upper end 4b is an upper end of the stopper 4.

The eaves portions 6 are provided to cover at least a part of the peripheral edge portion 11c of the microneedle device 11 when the microneedle device 11 supported by the stopper 4 is seen from the side of the top portion 22 in the height direction D1. As an example, the eaves portions 6 cover a part of the peripheral edge portion 11c. As an example, the eaves portions 6 are provided partially along the peripheral edge portion 11c so that other portions of the peripheral edge portion 11c are visible when the microneedle device 11 is seen from the side of the top portion 22 in the height direction D1. In the applicator 1E, the eaves portions 6 are provided at three locations along the peripheral edge portion 11c.

Next, a series of operations until the microneedle device 11 is applied to the skin when the applicator 1E is used will be described. The operation is the same as the operation in the applicator 1A described with reference to FIG. 4. In other words, as the user pushes the switch 3 toward the skin, the microneedle device 11 passes over the stopper 4 and reaches the skin. When the microneedle device 11 passes over the stopper 4, the second side portion 24 on which the stopper 4 is provided bends toward the first side portion 23, and thus the microneedle device 11 smoothly moves downward. In the applicator 1E, the stopper 4 is located closer to a vicinity of the free end 24a than the fixed end 24b of the second side portion 24. Since the second side portion 24 becomes more likely to bend toward the first side portion 23 as it approaches the free end 24a, the stopper 4 is more likely to move toward the first side portion 23.

### [Application Kit According to Fifth Example]

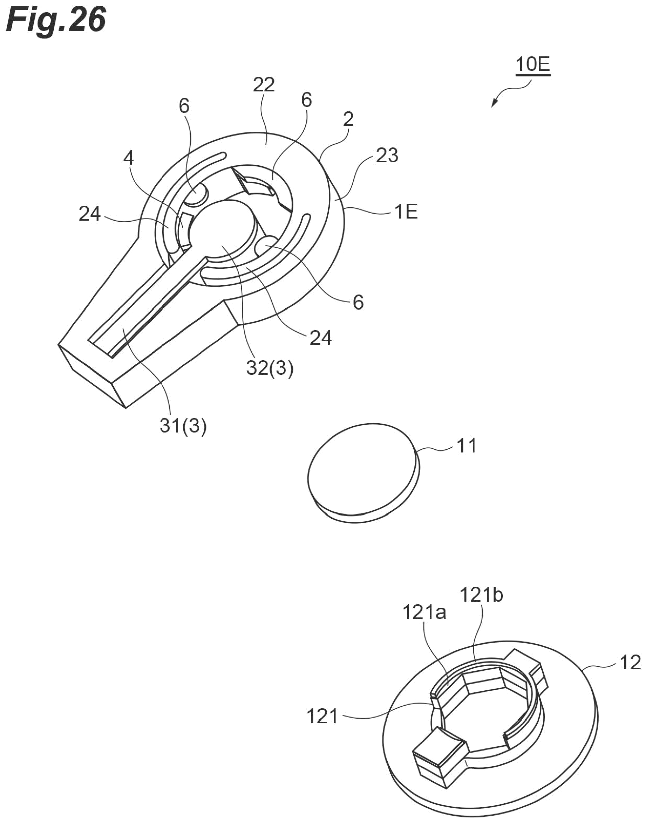

With reference to FIG. 26, a configuration of an application kit 10E according to the fifth example will be described. FIG. 26 is a perspective view of the application kit 10E seen from above.

The stage 12 is an article for arranging the microneedle device 11 in the applicator 1E. The stage 12 has a columnar portion 121 that extends in the height direction D1 to support the microneedle device 11. The columnar portion 121 is provided partially along the peripheral edge portion 11c of the microneedle device 11. The columnar portion 121 has a top surface 121a that supports the microneedle device 11 and a protrusion 121b that protrudes from the top surface 121a in the height direction D1. The top surface 121a is a surface that faces the microneedle device 11. As an example, the top surface 121a supports the peripheral edge portion 11c and does not come into contact with the platform surface 11d. The protrusion 121b is located outside the microneedle device 11 when the microneedle device 11 supported by the columnar portion 121 is seen from the side of the microneedle device 11 in the height direction D1. The protrusion 121b may or may not be in contact with the side surface 11e of the microneedle device 11.

In the application kit 10E, a process until the microneedle device 11 disposed on the stage 12 is disposed into the applicator 1E is the same as the process in the application kit 10A described with reference to FIG. 7.

### [Applicator According to Sixth Example]

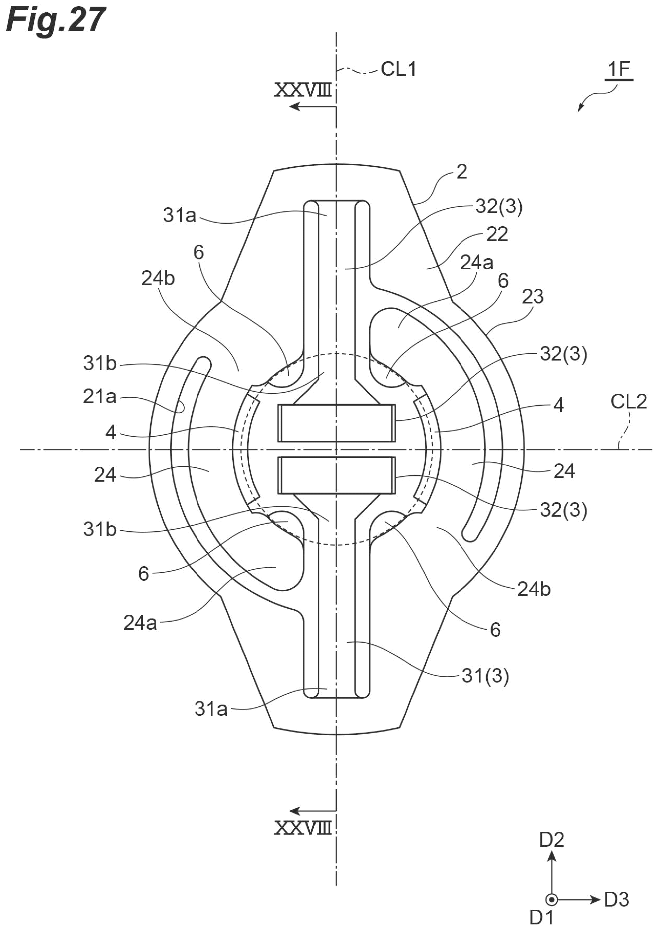

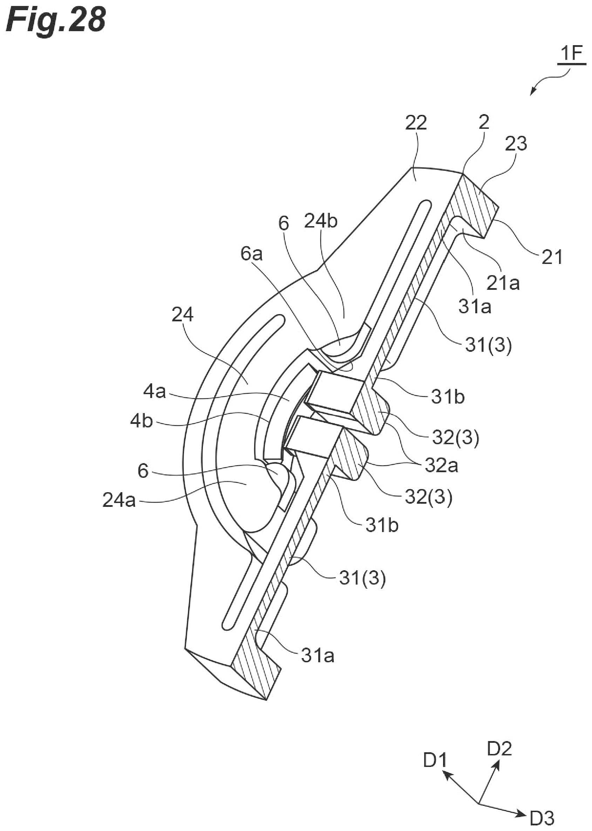

Referring to FIGS. 27 and 28, a structure of an applicator 1F according to a sixth example will be described. FIG. 27 is a plan view of the applicator 1F seen from above. FIG. 28 is a perspective cross-sectional view along line XXVIII-XXVIII shown in FIG. 27. FIGS. 27 and 28 show the applicator 1F in the natural state.

The applicator 1F includes a main body 2, at least one switch 3, at least one stopper 4, and at least one eaves portion 6. A material of the applicator 1F is the same as that of the applicator 1A, for example. A configuration of the microneedle device 11 set in the applicator 1F is the same as that of the microneedle device 11 set in the applicator 1A.

The main body 2 is a substantially rhombic-shaped structure that accommodates each component, such as the switch 3. The main body 2 has a bottom portion 21, a top portion 22, a first side portion 23 (a side portion) and a second side portion 24. The bottom portion 21 is a portion that comes into contact with the skin when the applicator 1F is placed on the skin to apply the microneedle device 11 to the skin. The bottom portion 21 has a substantially rhombic shape. An opening 21a for inserting the microneedle device 11 into the main body 2 is formed in a substantially center of the bottom portion 21. The top portion 22 is a portion opposite to the bottom portion 21 and is thus located away from that skin when the applicator 1F is placed on the skin.

Definitions of height direction D1, lateral direction D2, and width direction D3 used in the following description are the same as the definitions of the height direction D1, the lateral direction D2, and the width direction D3 used in the description of the first example. Further, the side of the top portion 22 in the height direction D1 may be referred to as the upper side of the applicator 1F, and the side of the bottom portion 21 in the height direction D1 may be referred to as the lower side of the applicator 1F.

The first side portion 23 extends in the height direction D1 and defines an outer circumference of the main body 2. As an example, the first side portion 23 extends in the circumferential direction of the bottom portion 21 and in the height direction D1 from the entire outer edge of the bottom portion 21. A top surface of the first side portion 23 may be present or function as the top portion 22. As an example, in order to improve user convenience, a height of a portion of the first side portion 23 from the bottom portion 21 (for example, a portion on the side of a fixed end 31a of an arm 31) may be lower than a height of the other portion of the first side portion 23 from the bottom portion 21.

The second side portion 24 is connected to the first side portion 23 inside the first side portion 23. The second side portion 24 extends in an arc shape so that a gap is formed between the second side portion 24 and the first side portion 23. The second side portion 24 extends along the peripheral edge portion 11c of the microneedle device 11 in the set state. Since a gap is formed between the entire surface of the second side portion 24 and the first side portion 23, the second side portion 24 can bend toward the first side portion 23.

As an example, the main body 2 is formed to be point-symmetrical with respect to the center of the main body 2. Each of the bottom portion 21, the top portion 22, and the first side portion 23 is formed to be line-symmetrical with respect to the imaginary center line CL1 parallel to the lateral direction D2. The second side portion 24 is formed to be point-symmetrical with respect to the center of the main body 2.

The switch 3 is a component that is operated by the user to push the microneedle device 11 toward the skin. As described above, the applicator 1F includes one or more switches 3, for example, two switches 3. The two switches 3 are located to be line-symmetrical with respect to an imaginary center line CL2 parallel to the width direction D3 of the microneedle device 11 in the set state. Each of the switches 3 has an arm 31 and a piston 32. The arm 31 is a rectangular plate-shaped portion mounted on the first side portion 23 to extend in the lateral direction D2. Therefore, the lateral direction D2 may also be referred to as an extension direction of the arm 31. The arm 31 has a fixed end 31a mounted on the first side portion 23, and a free end 31b located on the side opposite to the fixed end 31a. A free end 24a of the second side portion 24 is located near the free end 31b. The arm 31 can move in an arc shape in the height direction D1 with the fixed end 31a as a fulcrum, and this movement allows the free end 31b to move in the height direction D1. The arm 31 may be formed of a member or material that generates an elastic force, and may be, for example, an elastic member such as a leaf spring. Alternatively, the arm 31 may be a non-elastic member or a flexible member. The piston 32 is a plate-shaped portion mounted on the free end 31b. The piston 32 has a piston surface 32a which is a surface facing the skin. In one example, the switch 3 is formed to be line-symmetrical with respect to the center line CL1. As an example, the arm 31 extends parallel to the lateral direction D2 when seen in the width direction D3. The arm 31 may extend to be inclined with respect to the lateral direction D2 when seen in the width direction D3.

In the set state, the microneedle device 11 is located between the piston 32 and the bottom portion 21. The microneedle device 11 is disposed in the main body 2 so that the microneedle device 11 is located between the piston 32 and the skin when the bottom portion 21 comes into contact with the skin. In other words, it can be said that the piston 32, the microneedle device 11, and the bottom portion 21 (the skin) are arranged in this order in the height direction D1. The piston 32 is pushed in by the user toward the skin when the microneedle device 11 is applied to the skin. In other words, the piston 32 is pushed in by the user to the side of the bottom portion 21 in the height direction D1. At this time, the arm 31 moves in an arc shape with the fixed end 31a as a fulcrum in conjunction with the movement of the piston 32. The piston surface 32a comes into contact with the microneedle device 11 when it is pushed in by the user toward the skin. The piston surface 32a may or may not be in contact with the microneedle device 11 in the set state.

When the applicator 1F is seen in the height direction D1, the entire switch 3 is located inside opening 21a. Thus, the switch 3 does not come into contact with bottom portion 21 when the piston 32 is pushed in by the user toward the skin.

When the applicator 1F is seen from the side of top portion 22 in the height direction D1, the top portion 22 of the main body 2 does not cover at least the piston 32 and does not cover the entire switch 3, for example. The applicator 1F does not have a portion that covers the piston 32 from the side of the top portion 22 and does not have a portion that covers the entire switch 3 from the side of the top portion 22, for example. Therefore, when the applicator 1F is seen from the side of the top portion 22 in the height direction D1, the piston 32 is exposed to the outside of the applicator 1F.

The stopper 4 is a component that supports the microneedle device 11 which is disposed in the main body 2 to be located between the piston 32 and the skin. As described above, the applicator 1F includes one or more stoppers 4, for example, two stoppers 4. The two stoppers 4 may be provided to be line-symmetrical with respect to the imaginary center line (the center line CL1) of the arm 31 in the extension direction of the arm 31 as a reference axis. As an example, the two stoppers 4 are located to sandwich the piston 32 in the width direction D3. The two stoppers 4 may be located on the center line CL2. The two stoppers 4 are provided along the outer edge of the microneedle device 11 in the set state. Each of the stoppers 4 protrudes from the inner surface of the second side portion 24 toward the inside of the main body 2. In the applicator 1F, the two stoppers 4 are provided in a substantially center of the second side portion 24.

The stopper 4 overlaps the opening 21a and does not overlap the switch 3 when seen in the height direction D1. Therefore, when the switch 3 moves, the protrusion 41 does not come into contact with the switch 3. The stopper 4 has a placement surface 4a on which the microneedle device 11 is disposed in the set state. The placement surface 4a is a surface facing upward and is inclined with respect to a plane orthogonal to the height direction D1. The placement surface 4a may be parallel to the plane orthogonal to the height direction D1.

As an example, the stopper 4 supports the peripheral edge portion 11c. The stopper 4 may support the platform surface 11d as long as it does not come into contact with the microneedles 11a.

When the arm 31 generates an elastic force, like an elastic member such as a leaf spring, the arm 31 deforms toward the top portion 22, and thus an elastic force is generated in the arm 31. As an example, the microneedle device 11 inserted into the main body 2 through the opening 21a pushes the piston 32 upward, thereby causing the arm 31 to deform toward the top portion 22. Alternatively, the arm 31 is deformed toward the top portion 22 by such an insertion operation by the user. The switch 3, for example, the piston 32, transmits the elastic force generated in the arm 31 to the microneedle device 11 supported by the stopper 4. At this time, the switch 3 comes into contact with the microneedle device 11. The arm 31 may come into contact with the microneedle device 11, or the piston surface 32a may come into contact with the microneedle device 11. The stopper 4 supports the microneedle device 11 against the elastic force. In this way, the microneedle device 11 is gripped by the switch 3 and the stopper 4 and thus supported against the elastic force.

The eaves portion 6 is a component that stops the movement of the microneedle device 11 within the main body 2 so that the microneedle device 11, which is inserted into the main body 2 through the opening 21a and moves toward the top portion 22, does not pop out above the main body 2. As described above, the applicator 1F has one or more eaves portions 6, for example, four eaves portions 6. As an example, the four eaves portions 6 are provided to be line-symmetrical with respect to the imaginary center line (the center line CL1) of the arm 31 in the extension direction of the arm 31 as the reference axis. Each of the eaves portions 6 is provided at the free end 24a or the fixed end 24b of the second side portion 24. The eaves portions 6 protrude from the second side portion 24 toward the inside of the main body 2 when seen in the height direction D1. The eaves portions 6 overlap the opening 21a and do not overlap the switch 3 when seen in the height direction D1. Thus, when the switch 3 moves, the eaves portions 6 do not come into contact with the switch 3.

The eaves portions 6 are provided closer to the top portion 22 than the placement surface 4a of the stopper 4 in the height direction D1. In other words, the eaves portions 6 are located above the stopper 4. A distance between lower ends 6a of the eaves portions 6 and the upper end 4b of the placement surface 4a in the height direction D1 is greater than the thickness of the peripheral edge portion 11c of the microneedle device 11. From the viewpoint of holding the microneedle device 11 more stably, the distance between the lower ends 6a of the eaves portions 6 and the upper end 4b of the placement surface 4a may be the same as or smaller than the thickness of the peripheral edge portion 11c of the microneedle device 11. The lower ends 6a are ends of the eaves portions 6 that are located on the side of the bottom portion 21 in the height direction D1. The upper end 4b is an end of the placement surface 4a that is located on the side of the top portion 22 in the height direction D1. In the applicator 1F, the upper end 4b is an upper end of the stopper 4.

The eaves portions 6 are provided to cover at least a part of the peripheral edge portion 11c of the microneedle device 11 when the microneedle device 11 supported by the stopper 4 is seen from the side of the top portion 22 in the height direction D1. As an example, the eaves portions 6 cover a part of the peripheral edge portion 11c. As an example, the eaves portions 6 are provided partially along the peripheral edge portion 11c so that other portions of the peripheral edge portion 11c are visible when the microneedle device 11 is seen from the side of the top portion 22 in the height direction D1. In the applicator 1F, the eaves portions 6 are provided at four locations along the peripheral edge portion 11c.

A series of operations until the microneedle device 11 is applied to the skin when the applicator 1F is used are the same as those in the applicator 1A described with reference to FIG. 4. In other words, as the user pushes the switch 3 toward the skin, the microneedle device 11 passes over the stopper 4 and reaches the skin. When the microneedle device 11 passes over the stopper 4, the second side portion 24 on which the stopper 4 is provided bends toward the first side portion 23, and thus the microneedle device 11 smoothly moves downward.

### [Application Kit According to Sixth Example]

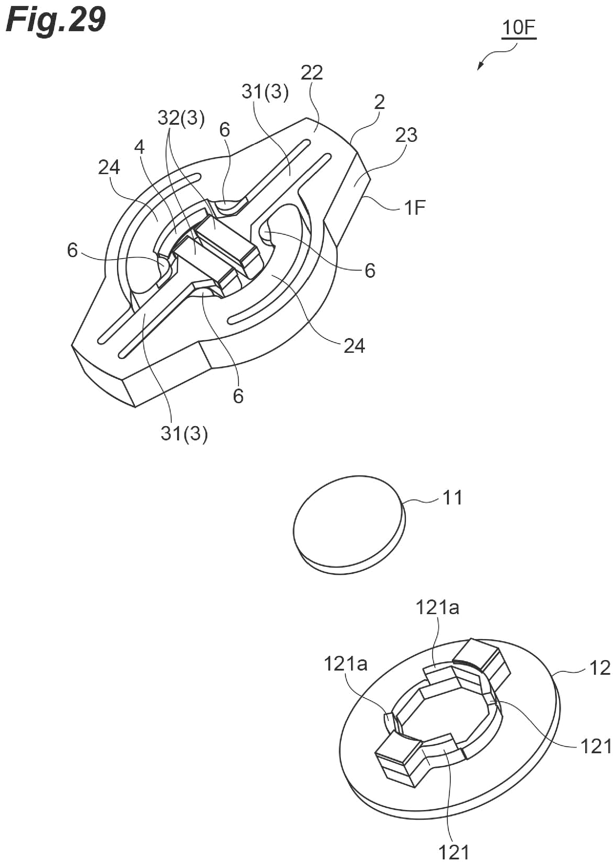

With reference to FIG. 29, a configuration of an application kit 10F according to the sixth example will be described. FIG. 29 is a perspective view of the application kit 10F seen from above.

The stage 12 is an article for arranging the microneedle device 11 in the applicator 1F. The stage 12 has a columnar portion 121 that extends in the height direction D1 to support the microneedle device 11. The columnar portion 121 is provided partially along the peripheral edge portion 11c of the microneedle device 11. The columnar portion 121 has a top surface 121a that supports the microneedle device 11. The top surface 121a is a surface that faces the microneedle device 11. As an example, the top surface 121a supports the peripheral edge portion 11c and does not come into contact with the platform surface 11d.

In the application kit 10F, a process until the microneedle device 11 disposed on the stage 12 is disposed into the applicator 1F is the same as the process in the application kit 10A described with reference to FIG. 7.

### [Action and Effect]

In the applicators 1A to 1F, the microneedle device 11 disposed in the main body 2 to be located between the piston 32 and the skin is supported by the stopper 4. Thus, the piston 32 can come into stable contact with the microneedle device 11 when the user pushes the piston 32 toward the skin. As a result, the microneedle device 11 is pushed securely toward the skin. Thus, the microneedle device 11 can be properly applied to the skin.

In the applicators 1A to 1F, the switch 3 transmits an elastic force generated by the deformation of the arm 31 toward the top portion 22 to the microneedle device 11, the stopper 4 supports the microneedle device 11 against the elastic force, and the microneedle device 11 is gripped by the switch 3 and the stopper 4 and thus supported against the elastic force. Thus, the microneedle device 11 can be stably held.

In the applicators 1A to 1F, the two stoppers 4 are provided to be line-symmetrical with respect to the imaginary center line (the center line CL1) of the arm 31 in the extension direction of the arm 31 as the reference axis. Thus, when the piston 32 comes into contact with the microneedle device 11, a pushing force of the user acts on the two stoppers 4 almost uniformly. Therefore, it becomes easier to push the microneedle device 11 straight into the skin, and as a result, the microneedle device 11 can be properly applied to the skin.

In the applicators 1A to 1E, the two stoppers 4 are provided closer to the arm 31 than the end portion of the piston 32 on the imaginary center line (the center line CL1) of the arm 31 in the extension direction of the arm 31. When the stopper 4 supports the microneedle device 11 against the elastic force of the arm 31, the elastic force is easily transmitted to the stopper 4. Thus, the microneedle device 11 can be held more stably.

The applicators 1A to 1D are provided with the ribs 5 which are located at different positions from the stopper 4 and are in contact with the side surface 11e of the microneedle device 11 that is supported by the stopper 4. Thus, the microneedle device 11 can be stably held.

In the applicators 1A to 1C, the microneedle device 11 has the main surface 11b on which the microneedles 11a are provided, and the rib 5 comes into contact with the side surface 11e without coming into contact with the main surface 11b of the microneedle device 11 supported by the stopper 4. Thus, the rib 5 can be prevented from coming into contact with the microneedles 11a, and as a result, the microneedles 11a can be prevented from breaking.

In the applicators 1A to 1D, the rib 5 is located on the imaginary center line (the center line CL1) of the arm 31 in the extension direction of the arm 31. Thus, when the microneedle device 11 is applied to the skin, the pushing force of the user is likely to act on the rib 5 straight along the center line, and as a result, it is easier to push the microneedle device 11 into the skin.

In the applicators 1A to 1F, the eaves portions 6 are provided to cover at least a part of the peripheral edge portion 11c of the microneedle device 11. Thus, when the microneedle device 11 moves towards the top portion 22, the eaves portions 6 stop the movement of the microneedle device 11 within the main body 2 . Thus, the microneedle device 11 can be set reliably in the applicator 1A.

The above actions and effects will be supplemented below. In the applicators 1A to 1F, the top portion 22 of the main body 2 does not cover the switch 3 when the applicators 1A to 1F are seen from the side of the top portion 22 in the height direction D1. Therefore, in a case in which the eaves portions 6 are not provided, when the microneedle device 11 is disposed inside the applicators 1A to 1F from below the applicator 1A to 1F, there is a risk that the microneedle device 11 will pop out above the applicator 1A to 1F. However, in the applicators 1A to 1F, since the eaves portions 6 are provided, it is possible to prevent the microneedle device 11 from popping out above the applicators 1A to 1F.

In the applicators 1A to 1F, the switch 3 transmits the elastic force generated by the deformation of the arm 31 toward the top portion 22 to the microneedle device 11, and the stopper 4 supports the microneedle device 11 against the elastic force. When the arm 31 is deformed toward the top portion 22 and the microneedle device 11 in contact with the switch 3 moves toward the top portion 22, the eaves portions 6 stop the movement of the microneedle device 11 toward the top portion 22. Thus, the microneedle device 11 can be held stably, and when the user operates the switch 3, the microneedle device 11 can be prevented from popping out above the applicators 1A to 1F.

In the applicators 1A to 1F, the eaves portions 6 cover a part of the peripheral edge portion 11c, and the eaves portions 6 are provided partially along the peripheral edge portion 11c so that the other portions of the peripheral edge portion 11c are visible when the microneedle device 11 supported by the stopper 4 is seen from the side of the top portion 22 in the height direction D1. Thus, even when the eaves portions 6 are provided, the user can easily visually confirm a position of the microneedle device 11.

In the applicators 1A to 1F, the eaves portions 6 are formed to be line-symmetrical with respect to the imaginary center line (the center line CL1) of the arm 31 in the extension direction of the arm 31 as the reference axis. Thus, the eaves portion 6 can more reliably stop the movement of the microneedle device 11 toward the top portion 22.

The applicators 1A to 1C include the recovery mechanism 7 that holds the microneedle device 11 at least when the microneedle device 11 is applied to the skin by an operation of the switch 3 by the user. Thus, when the applicators 1A to 1C are moved away from the skin, the microneedle device 11 applied to the skin is held by the recovery mechanism 7 and moved away from the skin together with the applicators 1A to 1C. Thus, the microneedle device 11 applied to the skin can be easily recovered.

In the applicators 1A to 1C, the recovery mechanism 7 has two gripping claws 71 that grip the microneedle device 11. Thus, the configuration in which the recovery mechanism 7 holds the microneedle device 11 can be easily realized.

In the applicators 1A and 1B, each of the two gripping claws 71 is provided on the piston 32 to be located outside of the piston surface 32a of the piston 32. Thus, in the state in which the microneedle device 11 is in contact with the piston surface 32a, the gripping claws 71 can easily grip the microneedle device 11.

In the applicators 1A and 1B, the two gripping claws 71 grip the microneedle device 11 before the microneedle device 11 reaches the skin in response to the piston 32 moving toward the skin. Thus, the microneedle device 11 can be reliably held.

The applicators 1A and 1B are provided with the stopper 4 that supports the microneedle device 11 disposed in the main body 2 to be located between the piston 32 and the skin. Thus, when the two gripping claws 71 grip the microneedle device 11, the microneedle device 11 continues to be supported by the stopper 4. Thus, the two gripping claws 71 can reliably grip the microneedle device 11.

In the applicators 1A to 1C, the two gripping claws 71 do not grip the microneedle device 11 from the time the microneedle device 11 is inserted into the main body 2 toward the piston 32 until it is supported by the stopper 4. Thus, even when the gripping claws 71 are provided, the microneedle device 11 can be easily disposed inside the main body 2.

In the applicator 1C, the recovery mechanism 7 is a plurality of L-shaped members located outside the piston 32, and each of the plurality of L-shaped members has the horizontal portion 72 that extends in the width direction D3, the vertical portion 73 that extends from the inner end 72a of the horizontal portion 72 located inside the main body 2 to the top portion 22 in the height direction D1, and the gripping claws 71 provided at the inner end 72a. By providing such a plurality of L-shaped members outside the piston 32, the microneedle device 11 can be gripped by the plurality of gripping claws 71 without interfering with the movement of the piston 32 which acts directly when the microneedle device 11 is applied to the skin.

In the applicator 1C, the two gripping claws 71 grip the microneedle device 11 in response to the microneedle device 11 being applied to the skin. Thus, the microneedle device 11 applied to the skin can be more reliably gripped.

The applicator 1C includes the eaves portion 6 (the blocking portion) that is provided inside the main body 2 with respect to the upper end 73a of the vertical portion 73 located on the side of the top portion 22 and limits the extent to which the vertical portion 73 tilts toward the inside of the main body 2. Thus, since the vertical portion 73 is not excessively tilted toward the inside of the main body 2, the microneedle device 11 can be kept inside the main body 2.

In the application kits 10A to 10F, in response to the insertion of the columnar portion 121 supporting the microneedle device 11 into the main body 2 toward the piston 32, the microneedle device 11 enters between the piston 32 and the stopper 4 and is supported by the stopper 4. Thus, the microneedle device 11 can be easily disposed inside the applicators 1A to 1F from the outside of the applicators 1A to 1F. Thus, the microneedle devices 11 can be easily set in the applicators 1A to 1F.

In the application kits 10A and 10E, the columnar portion 121 has the top surface 121a that supports the microneedle device 11 and the protrusion 121b that protrudes from the top surface 121a, and the protrusion 121b is located outside the microneedle device 11 when the microneedle device 11 supported by the columnar portion 121 is seen from the side of the microneedle device 11 in the height direction D1. Thus, since the movement of the microneedle device 11 along the plane orthogonal to the height direction D1 is restricted, the microneedle device 11 can be set reliably in the applicators 1A and 1E.

In the application kits 10A to 10F, the microneedle device 11 has the main surface 11b on which one or more microneedles 11a are provided, the main surface 11b has the peripheral edge portion 11c that defines the outer edge of the microneedle device 11, and the platform surface 11d formed inside the peripheral edge portion 11c to be raised from the peripheral edge portion 11c and formed to support one or more microneedles 11a, and the columnar portion 121 has the top surface 121a that supports the peripheral edge portion 11c without coming into contact with the platform surface 11d. Thus, since the movement of the microneedle device 11 along the plane orthogonal to the height direction D1 is restricted, the microneedle device 11 can be set reliably in the applicators 1A to 1F. Further, it is possible to prevent the columnar portion 121 from coming into contact with the microneedles 11a.

### [Modified Example]

The technology according to the present disclosure has been described in detail above based on various examples. However, the present disclosure is not limited to the above examples. The technology according to the present disclosure can be modified in various ways without departing from the spirit thereof.

In the applicators 1A to 1C, the rib 5 may have a protrusion 51 that protrudes toward the end portion 32b of the piston 32 when seen in the height direction D1, as in the applicator 1D. In this case, in the applicators 1A to 1C, the microneedle device 11 can be held more stably.

The applicator 1A may further include two eaves portions 6 located on the same side of the piston 32 as the stopper 4. That is, the applicator 1A may include four eaves portions 6, and two eaves portions 6 and the other two eaves portions 6 may be located to sandwich the piston 32. In the applicator 1A, the four eaves portions 6 may be provided at the same position as the four eaves portions 6 provided in the applicator 1D. In this case, the microneedle device 11 can be set reliably by the applicator 1A.

In the applicator 1A, the stopper 4 extends from the bottom portion 21 in the height direction D1, but the stopper 4 may be provided to extend from the inner surface of the first side portion 23 toward the center of the main body 2.

### [Appendices]

As can be seen from the various examples above, the present disclosure includes the following aspects.

### (Appendix 1)

An applicator for applying a microneedle device to skin, including:
a main body including a bottom portion in contact with the skin, a top portion opposite to the bottom portion, and a side portion that extends in a height direction from the bottom portion toward the top portion;
a switch including an arm mounted on the side portion to extend in a lateral direction from the side portion toward a center of the main body, and a piston provided at a free end of the arm and pushed in by a user toward the skin; and
at least one stopper that supports the microneedle device disposed in the main body to be located between the piston and the skin.

### (Appendix 2)

The applicator as described in Appendix 1, wherein the switch transmits an elastic force generated by deformation of the arm toward the top portion to the microneedle device,
the at least one stopper supports the microneedle device against the elastic force, and
the microneedle device is supported against the elastic force by being gripped by the switch and the at least one stopper.

### (Appendix 3)

The applicator as described in Appendix 2 wherein the at least one stopper includes two stoppers, and
the two stoppers are provided to be line-symmetrical with respect to an imaginary center line of the arm in an extension direction of the arm as a reference axis.

### (Appendix 4)

The applicator as described in Appendix 3, wherein the two stoppers are provided closer to the arm than an end portion of the piston on the imaginary center line.

### (Appendix 5)

The applicator as described in any one of Appendices 1 to 4, further including a rib provided at a position different from the stopper and in contact with at least a side surface of the microneedle device supported by the stopper.

### (Appendix 6)

The applicator as described in Appendix 5, wherein the microneedle device includes a main surface on which one or more microneedles are provided, and
the rib comes into contact with the side surface without coming into contact with the main surface of the microneedle device supported by the stopper.

### (Appendix 7)

The applicator as described in Appendix 5 or 6, wherein the rib is located on an imaginary center line of the arm in an extension direction of the arm.

### Reference Signs List

1A to 1F Applicator, 2 Main body, 3 Switch, 4 Stopper, 5 Rib, 11 Microneedle device, 11a Microneedle, 11b Main surface, 11e Side surface, 21 Bottom portion, 22 Top portion, 23 First side portion (side portion), 31 Arm, 31b Free end, 32 Piston, 32b End portion, CL1 Center line, D1 Height direction, D2 Lateral direction

## Claims

1. An applicator for applying a microneedle device to skin, comprising:
a main body including a bottom portion in contact with the skin, a top portion opposite to the bottom portion, and a side portion that extends in a height direction from the bottom portion toward the top portion;
a switch including an arm mounted on the side portion to extend in a lateral direction from the side portion toward a center of the main body, and a piston provided at a free end of the arm and pushed in by a user toward the skin; and
at least one stopper that supports the microneedle device disposed in the main body to be located between the piston and the skin.

2. The applicator according to claim 1, wherein the switch transmits an elastic force generated by deformation of the arm toward the top portion to the microneedle device,
the at least one stopper supports the microneedle device against the elastic force, and
the microneedle device is supported against the elastic force by being gripped by the switch and the at least one stopper.

3. The applicator according to claim 2, wherein the at least one stopper includes two stoppers, and
the two stoppers are provided to be line-symmetrical with respect to an imaginary center line of the arm in an extension direction of the arm as a reference axis.

4. The applicator according to claim 3, wherein the two stoppers are provided closer to the arm than an end portion of the piston on the imaginary center line.

5. The applicator according to any one of claims 1 to 4, further comprising a rib provided at a position different from the stopper and in contact with at least a side surface of the microneedle device supported by the stopper.

6. The applicator according to claim 5, wherein the microneedle device includes a main surface on which one or more microneedles are provided, and
the rib comes into contact with the side surface without coming into contact with the main surface of the microneedle device supported by the stopper.

7. The applicator according to claim 5, wherein the rib is located on an imaginary center line of the arm in an extension direction of the arm.
